# EUROPEAN PATENT APPLICATION

(11) **EP 0 623 595 A1**
(43) Date of publication of application: **09.11.1994**
(21) Application number: 94106858.7
(22) Date of filing: 02.05.1994
(51) Int. Cl.: C07D 207/08, C07D 401/12, C07D 211/24, C07D 223/06, C07D 223/08, C07D 223/16, C07D 211/58, A61K 31/40, A61K 31/47, A61K 31/445

(54) **Guanidinyl- or amidinyl-substituted heterocyclic thrombin inhibitors**

(30) Priority: 03.05.1993 US 56279
(71) Applicant: BRISTOL-MYERS SQUIBB COMPANY, Princeton, NJ 08543-4000 (US)
(72) Inventor: Kimball, Spencer D., East Windsor, New Jersey (US); Hall, Steven E., Chapel Hill, North Carolina (US); Lau, Wan Fang, Lawrenceville, New Jersey (US)
(74) Representative: VOSSIUS & PARTNER

(57) **Abstract**

Guanidinyl- or amidinyl-substituted heterocyclic thrombin inhibitors are provided which have the structure 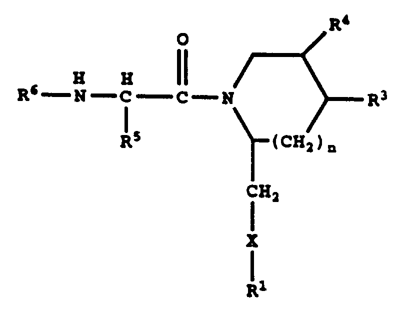
wherein n is 0, l or 2;
X is S, SO, SO₂ or O;
R¹ is -A-R², where A is an alkyl, alkenyl, or alkynyl chain of 2 to 6 carbon atoms and R² is guanidine, amidine, or amino; or
R¹ is -(CH₂)ₚ-A'-R^{2'} where R^{2'} is amidine and A' is an azacycloalkyl ring of 4 to 8 atoms, optionally substituted by alkyl, CO or halo; or
R¹ is -(CH₂)ₚ-A''-R^{2''},
wherein R^{2''} is guanidine, amidine or aminomethyl, and A'' is aryl or cycloalkyl;
and p, R³, R⁴, R⁵ and R⁶ are as defined herein.

## Description

The present invention relates to guanidinyl- or amidinyl-substituted heterocyclic compounds, which are thrombin inhibitors and thus useful in inhibiting formation of thrombi.

The guanidinyl- or amidinyl-substituted heterocyclic thrombin inhibitors of the invention have the structure I 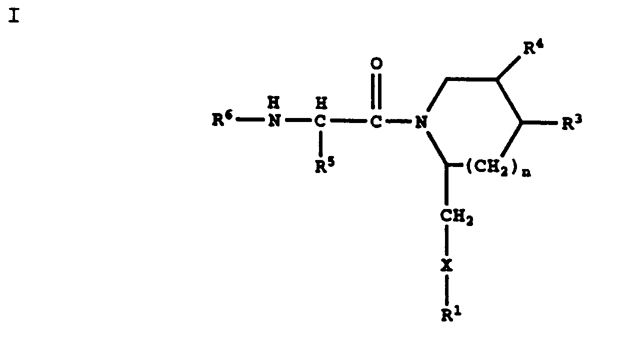
including all stereoisomers thereof, wherein n is 0, l or 2;
X is S, SO, SO₂ or O;
R¹ is -A-R², where A is an alkyl, alkenyl, or alkynyl chain of 2 to 6 carbon atoms and R² is guanidine, amidine, or amino; or
R¹ is -(CH₂)ₚ-A'-R^{2'} where R^{2'} is amidine and A' is an azacycloalkyl ring of 4 to 8 atoms, optionally substituted by alkyl, CO or halo as given by the structure 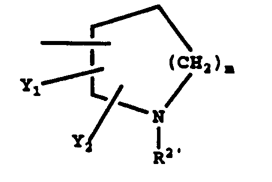
where p is 0, l or 2, m is 0, l, 2, 3 or 4, N and X are separated by at least two carbon atoms; and
Y₁, Y₂ are independently H, alkyl, halo or keto(oxo); or
R¹ is -(CH₂)ₚ-A''-R^{2''},
wherein R^{2''} is guanidine, amidine or aminomethyl, and A'' is aryl or cycloalkyl;
R³ and R⁴ are independently hydrogen, lower alkyl, cycloalkyl, aryl, hydroxy, alkoxy, keto(oxo), thioketal, thioalkyl, thioaryl, amino or alkylamino, or R³ and R⁴ together with the carbons to which they are attached form a cycloalkyl, aryl, or heteroaryl ring;
R⁵ is hydrogen, hydroxyalkyl, aminoalkyl, amidoalkyl, alkyl, cycloalkyl, aryl, arylalkyl, alkenyl, alkynyl, arylalkoxyalkyl, or an amino acid side chain, either protected or unprotected; and
R⁶ is hydrogen, 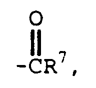
-SO₂R⁷ or -CO₂R⁷ (wherein R⁷ is lower alkyl, aryl, cycloheteroalkyl or heteroaryl);
including pharmaceutically acceptable salts thereof.

Preferred are compounds of formula I wherein X is S or SO₂, n is 0, R¹ is -CH₂(CH₂)_{q}-R², q is l, 2, 3 or 4; more preferably 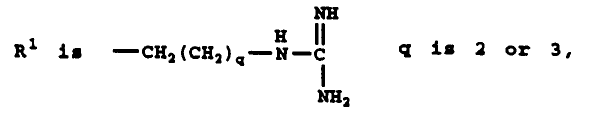
R³ and R⁴ are each H, R⁵ is H or -CH₂OH, and R⁶ is 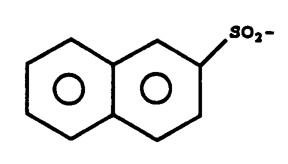
The term "lower alkyl" or alkyl" as employed herein by itself or as part of another group includes both straight and branched chain radicals of up to l8 carbons, preferably l to 8 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as well as such groups including l, 2 or 3 halo substituents, an aryl substituent, an alkylaryl substituent, a haloaryl substituent, a cycloalkyl substituent, an alkylcycloalkyl substituent, an alkenyl substituent, an alkynyl substituent, hydroxy or a carboxy substituent.

The term "cycloalkyl" by itself or as part of another group includes saturated cyclic hydrocarbon groups containing 3 to l2 carbons, preferably 3 to 8 carbons, which include cyclopropyl, cyclobutyl, cyclopentyl, cyclohexyl, cycloheptyl, cyclooctyl, cyclodecyl and cyclododecyl, any of which groups may be substituted with substituents such as halogen, lower alkyl, alkoxy and/or hydroxy groups.

The term "aryl" or "Ar" as employed herein by itself or as part of another group refers to monocyclic or bicyclic aromatic groups containing from 6 to l0 carbons in the ring portion, such as phenyl, or naphthyl. Aryl (or Ar), phenyl or naphthyl may include substituted aryl, substituted phenyl or substituted naphthyl, which may include l or 2 substituents on either the Ar, phenyl or naphthyl such as lower alkyl, cyano, amino, alkylamino, dialkylamino, nitro, carboxy, carboalkoxy, trifluoromethyl, halogen (Cl, Br, I or F), lower alkoxy, arylalkoxy, hydroxy, alkylthio, alkylsulfinyl, alkylsulfonyl, arylthio, arylsulfinyl and/or arylsulfonyl.

The term "aralkyl", "aryl-alkyl" or "aryl-lower alkyl" as used herein by itself or as part of another group refers to lower alkyl groups as discussed above having an aryl substituent, such as benzyl.

The term "lower alkoxy", "alkoxy" or aralkoxy" includes any of the above lower alkyl, alkyl or aralkyl groups linked to an oxygen atom.

The term "halogen" or "halo" as used herein by itself or as part of another group refers to chlorine, bromine, fluorine or iodine with chlorine being preferred.

The term "lower alkenyl" or "alkenyl" as employed herein by itself or as part of another group includes a carbon chain of up to l6 carbons, preferably 3 to l0 carbons, containing one double bond which will be separated from "N" or "X" by at least one saturated carbon moiety such as -(CH₂)_{q}-where q can be l to l4, such as 2-propenyl, 2-butenyl, 3-butenyl, 2-pentenyl, 4-pentenyl and the like, and may include a halogen substituent such as I, Cl, or F.

The term "lower alkynyl" or "alkynyl" as employed herein by itself or as part of another group includes a carbon chain of up to l6 carbons, preferably 3 to l0 carbons, containing one triple bond which will be separated from "N" or "X" by at least one saturated carbon moiety such as -(CH2)_{q'}-where q' can be l to l4, such as 2-propynyl, 2-butynyl, 3-butynyl and the like.

The term "heteroaryl" or heteroaromatic by itself or as part of another group refers to a 5- or 6-membered aromatic ring which includes l or 2 hetero atoms such as nitrogen, oxygen or sulfur, such as 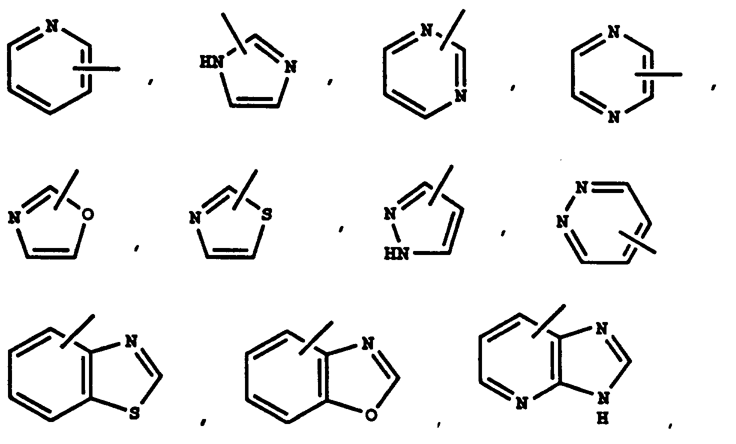 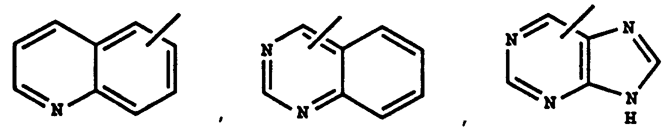
and the like. The heteroaryl rings may optionally be fused to aryl rings defined previously. The hetero-aryl rings may optionally include l or 2 substituents such as halogen (Cl, Br, F or CF₃), lower alkyl, lower alkoxy, carboxy, amino, lower alkylamino and/or dilower alkylamino.

The term "cycloheteroalkyl" as used herein refers to a 5-, 6- or 7-membered saturated ring which includes l or 2 hetero atoms such as nitrogen, oxygen and/or sulfur, such as 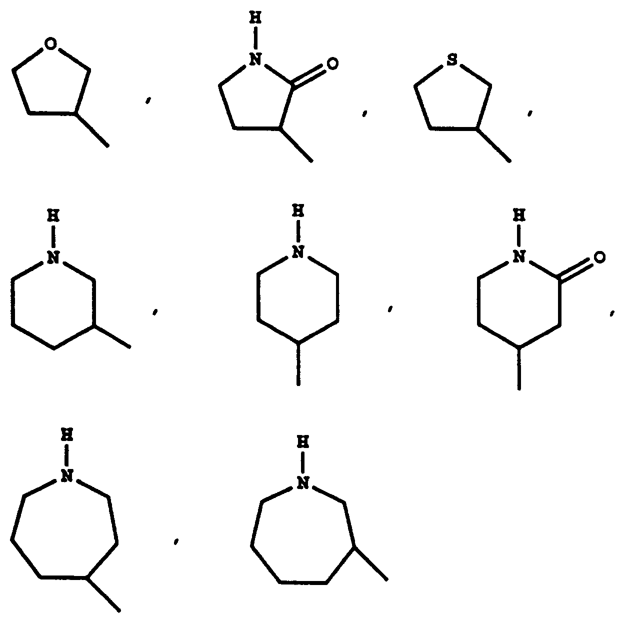
and the like.

The term "amino acid side chain" refers to any of the known alpha-amino acids such as arginine, histidine, alanine, glycine, lysine, glutamine, leucine, valine, serine, homoserine, allothreonine, naphthylalanine, isoleucine, phenylalanine and the like.

The compounds of formula I of the invention wherein X is S, SO, SO₂ or O may be prepared according to the following reaction sequences.

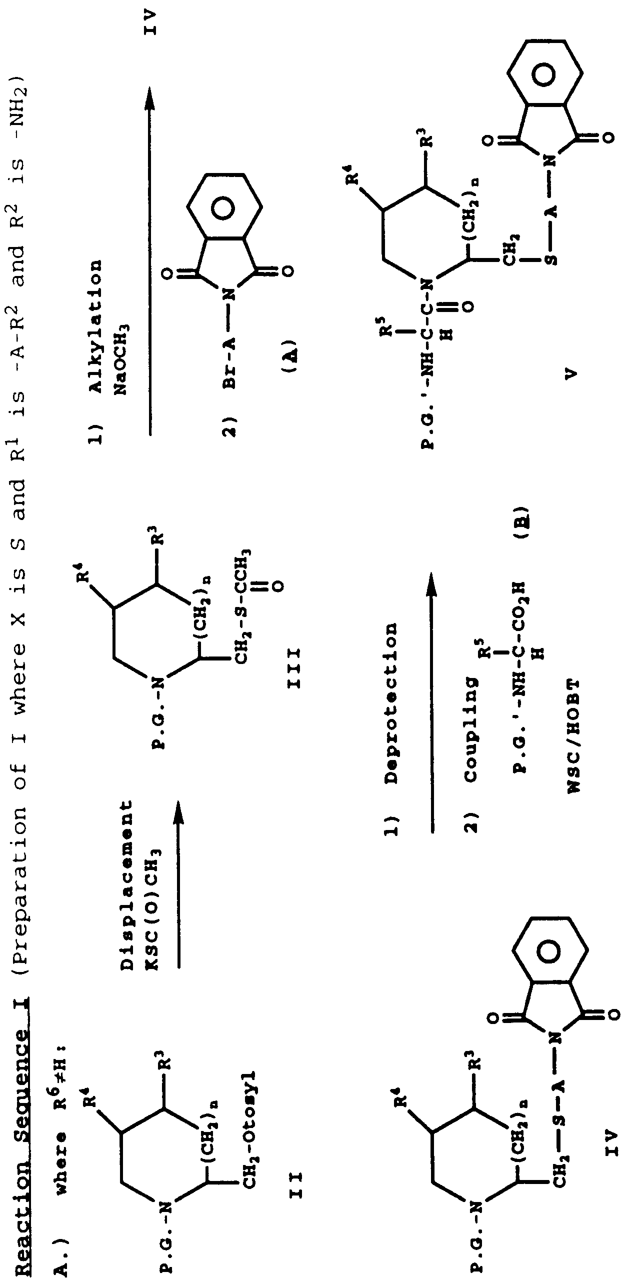

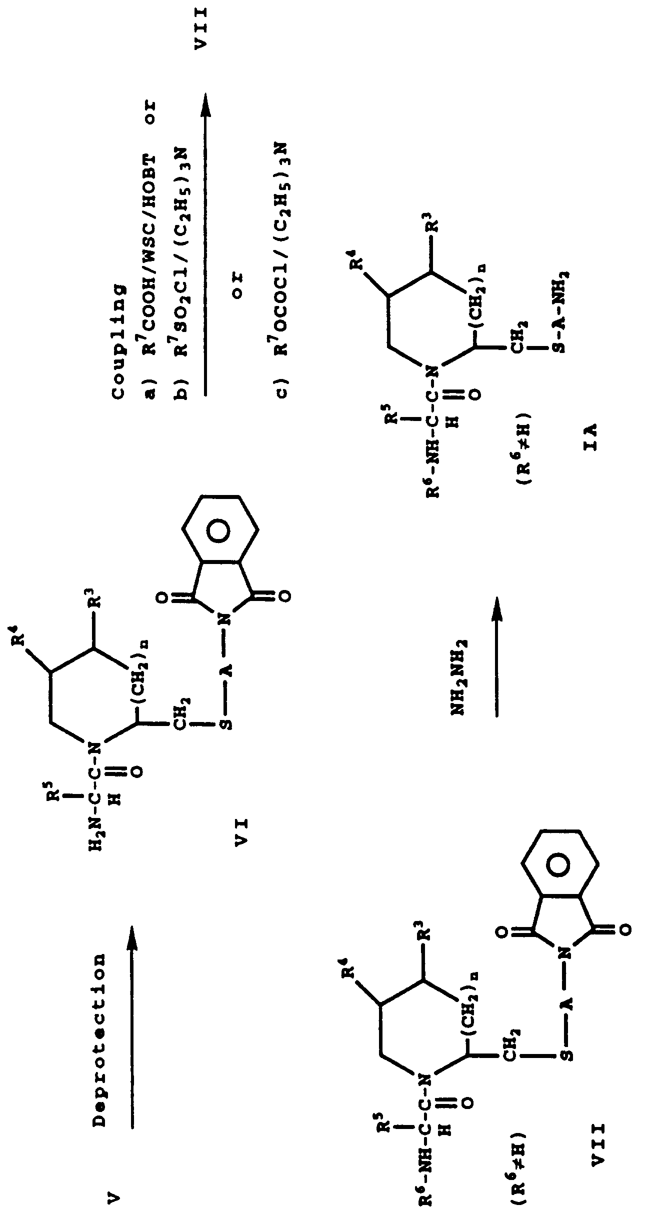

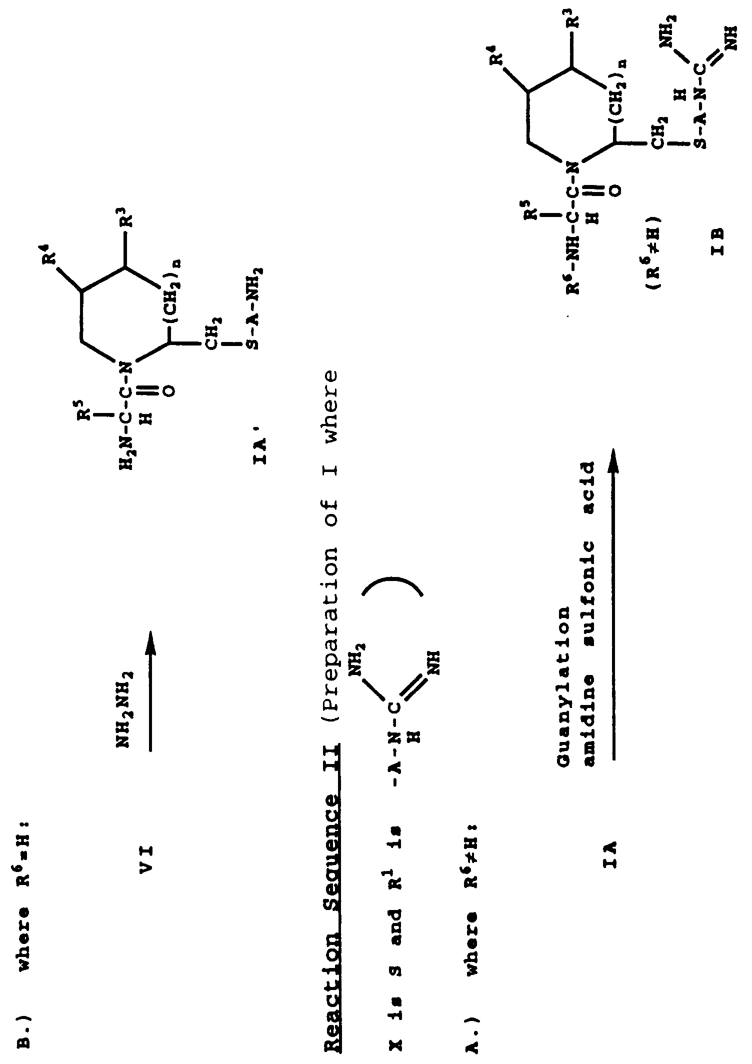

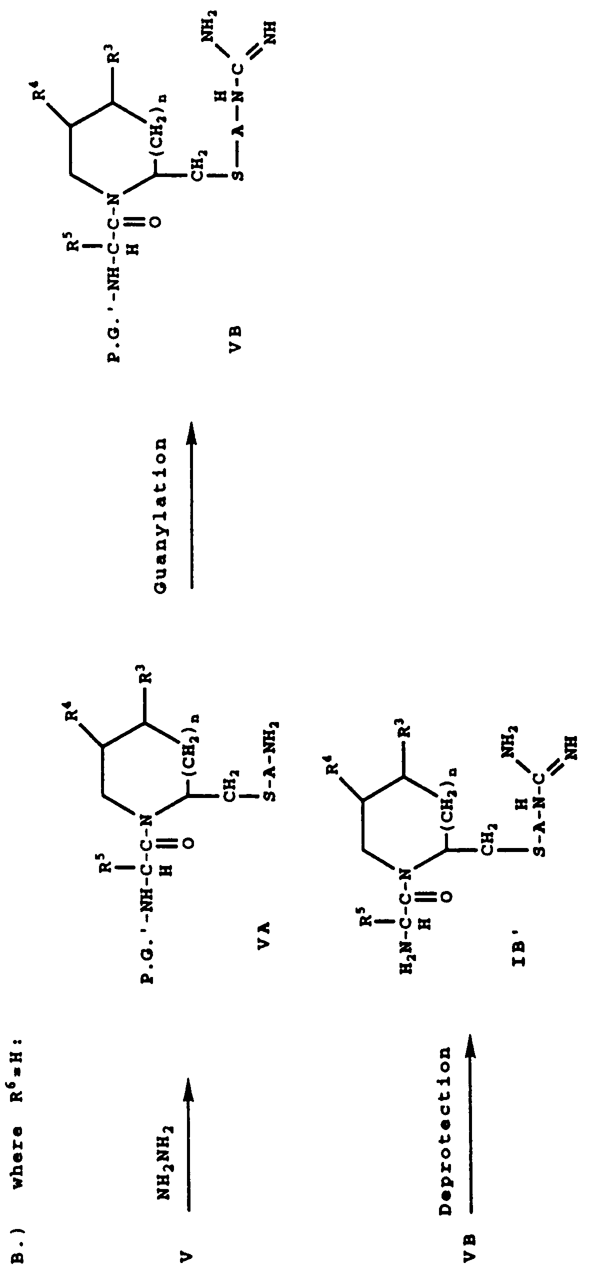

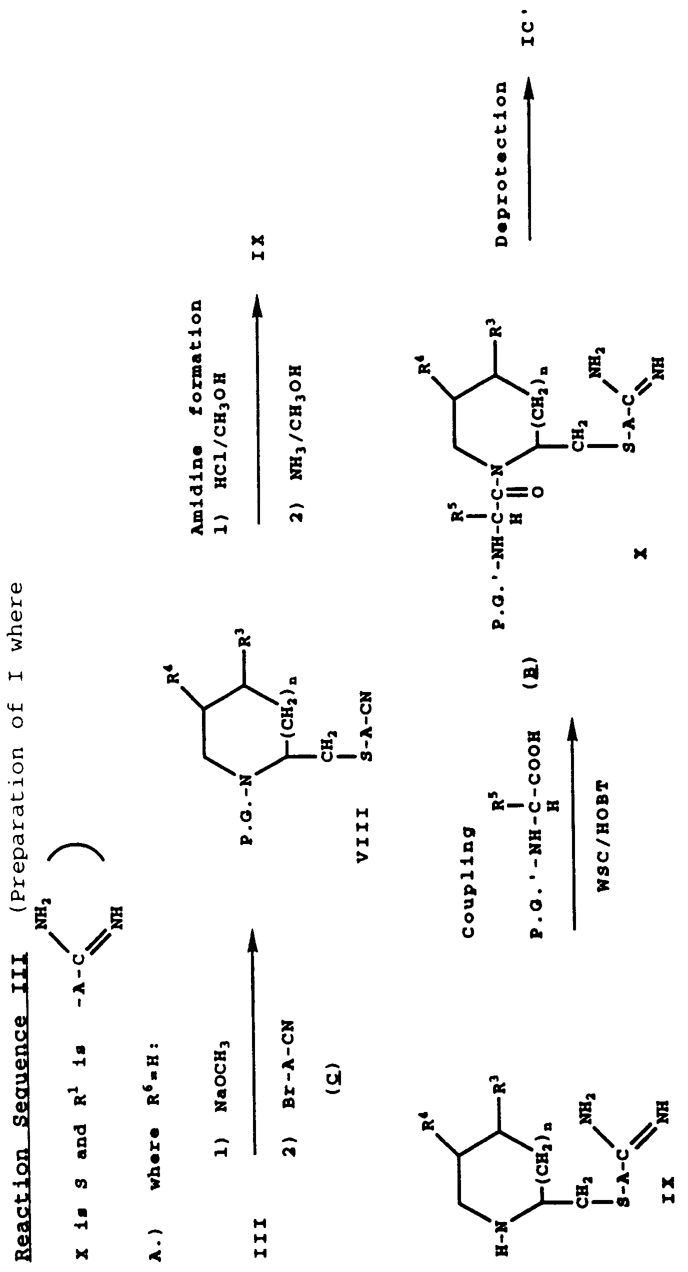

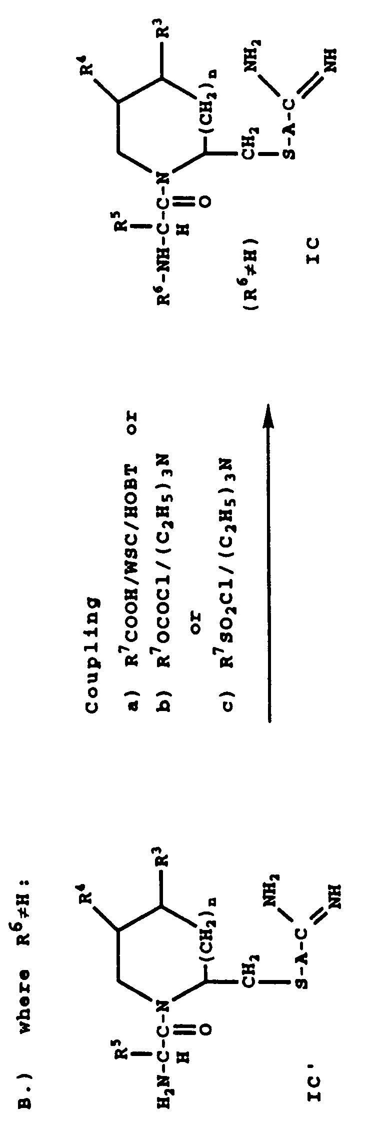

### Reaction Sequence IV (Preparation of I where X is SO or SO₂ and R¹ is -A-R²)

A.) where R⁶ may or may not be H and R² is amino: 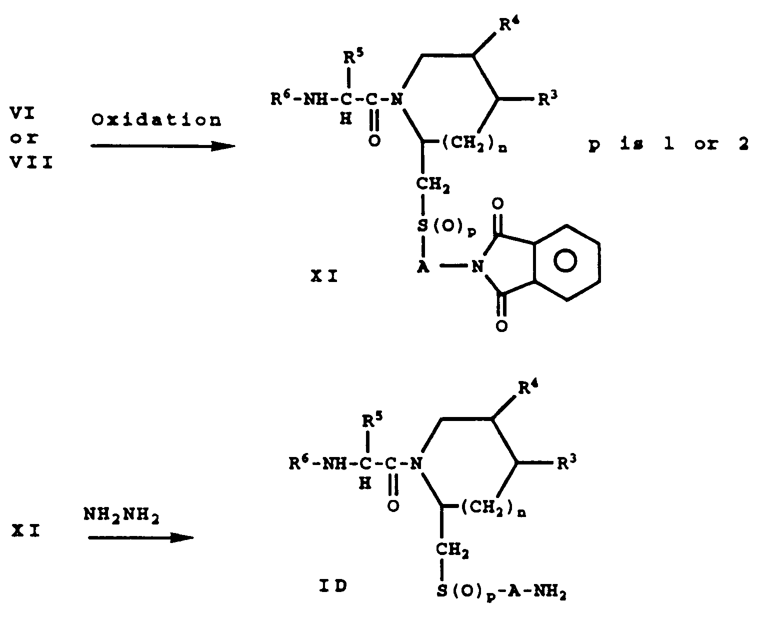
B1.) where R⁶≠H and R² is guanidinyl: 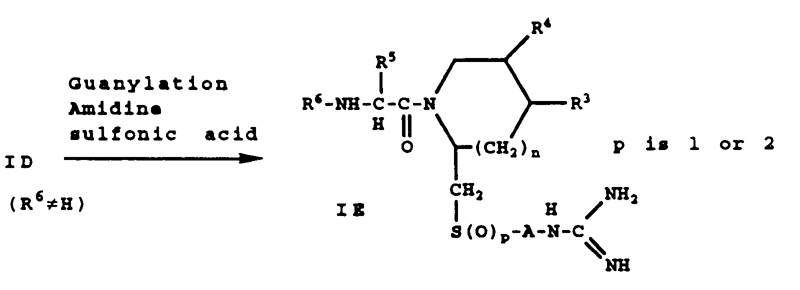
B2.) where R⁶ is H and R² is guanidinyl: 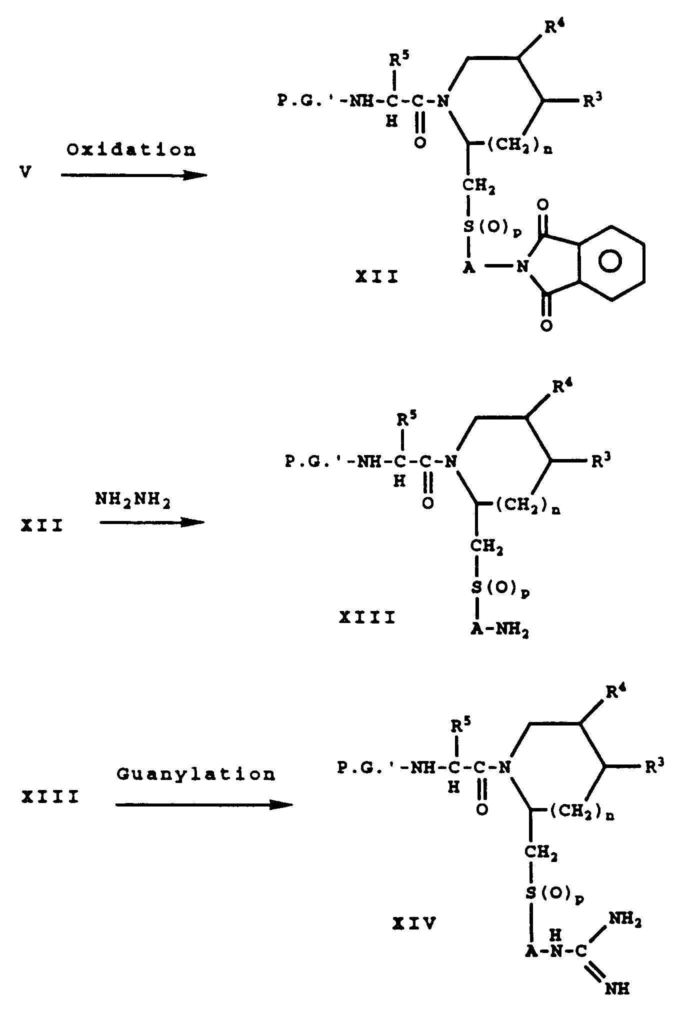 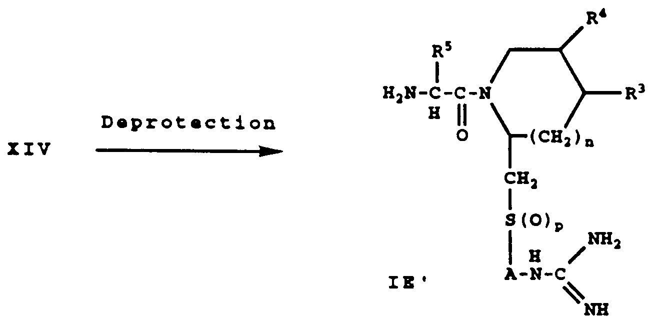
C.) where R⁶ may or may not be H and R² is amidinyl:

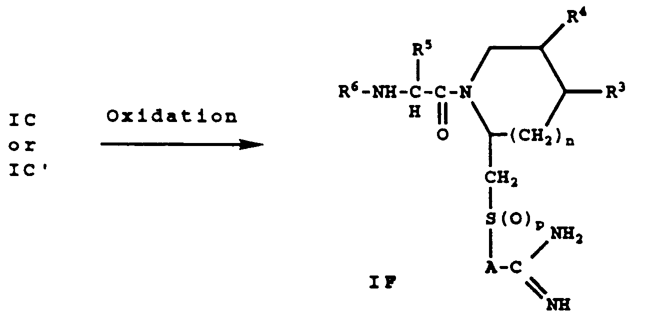

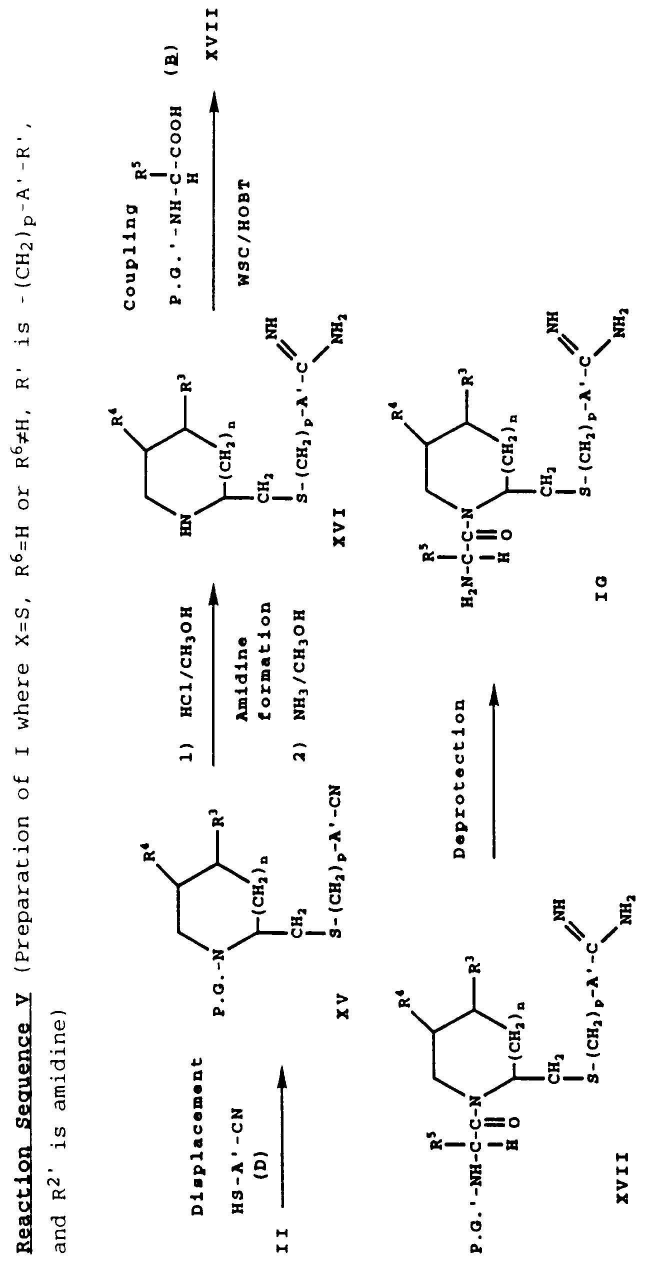

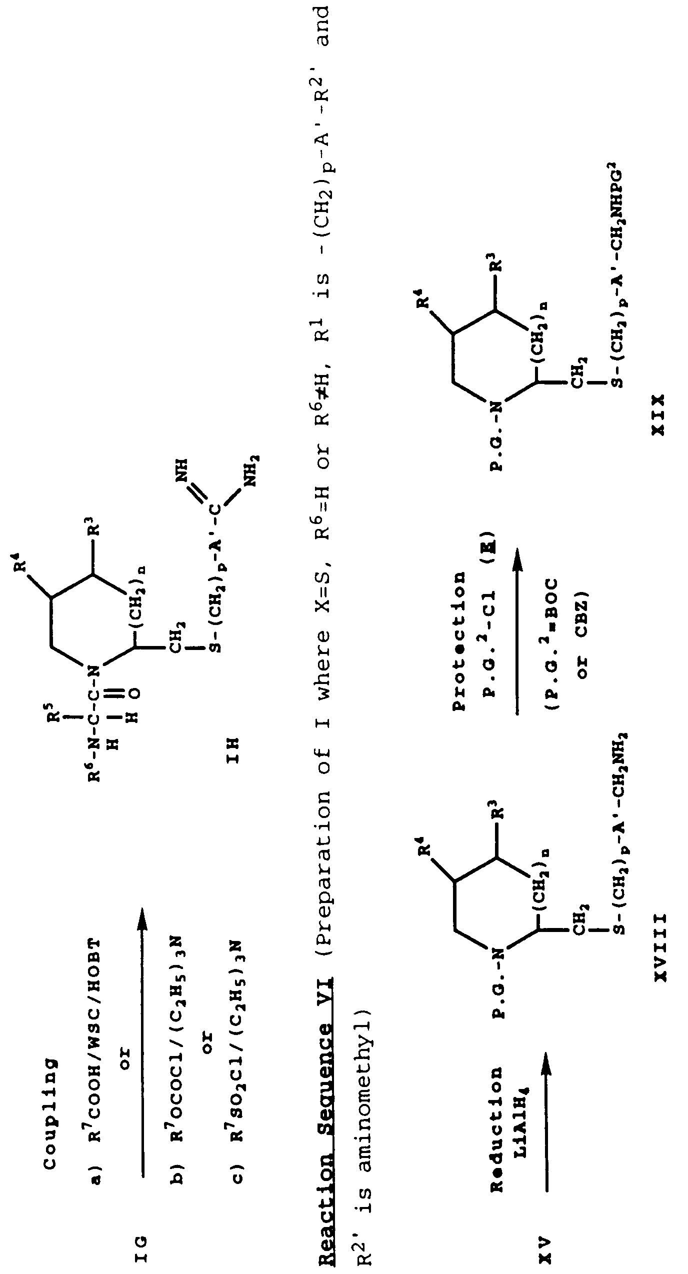

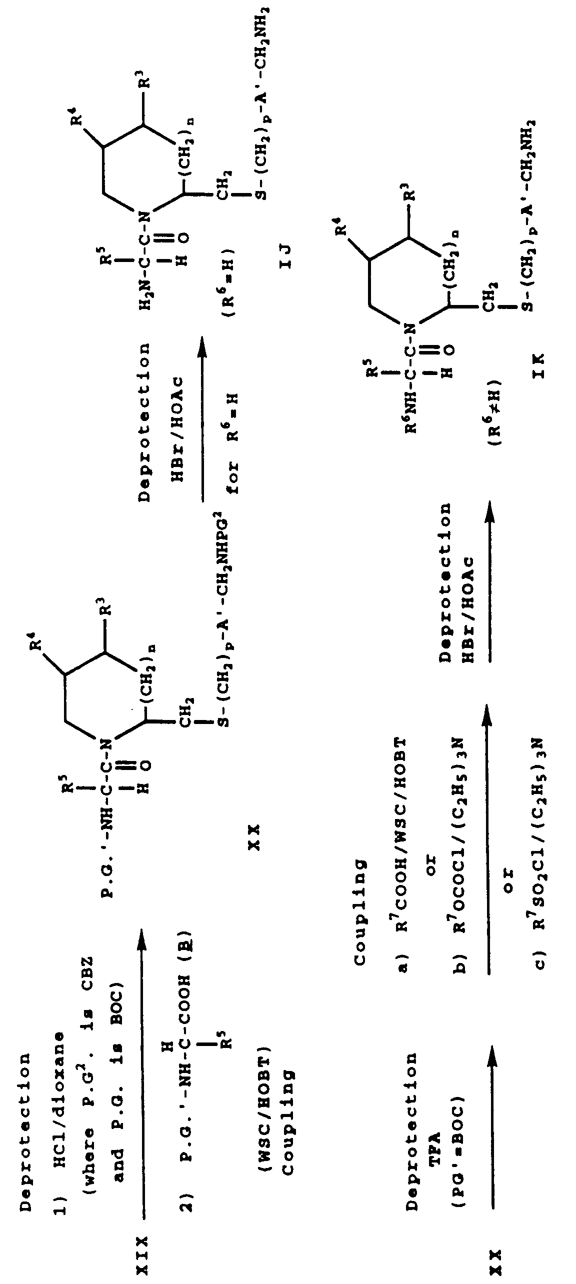

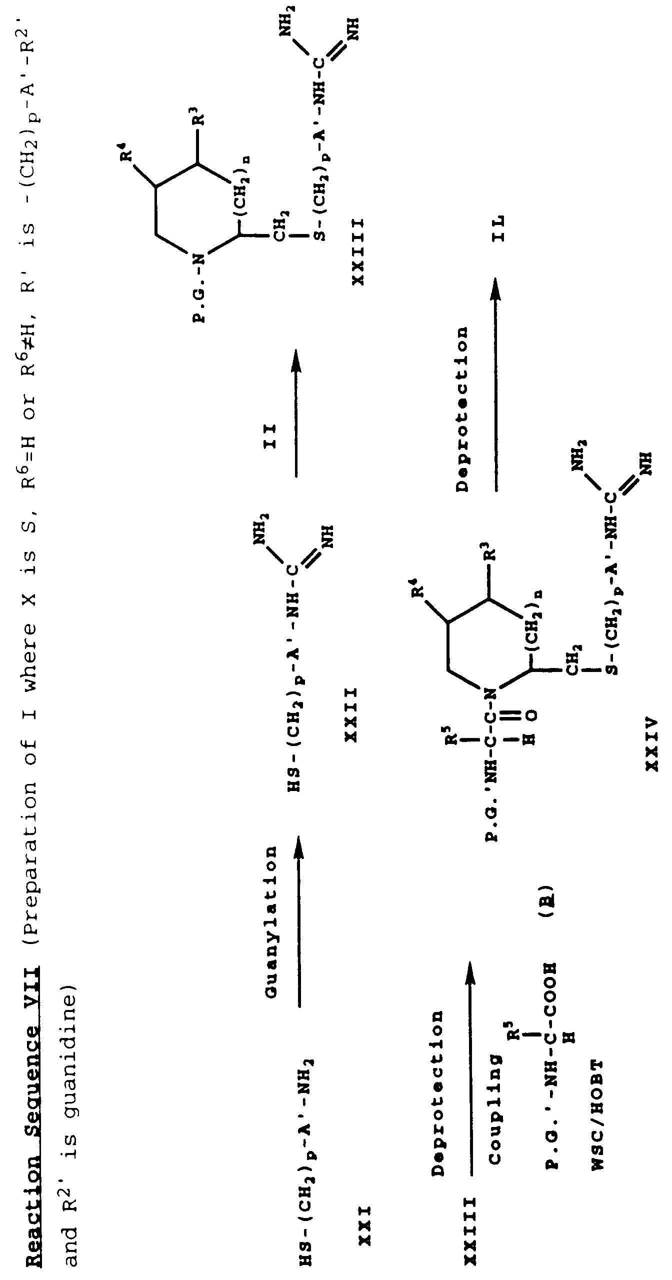

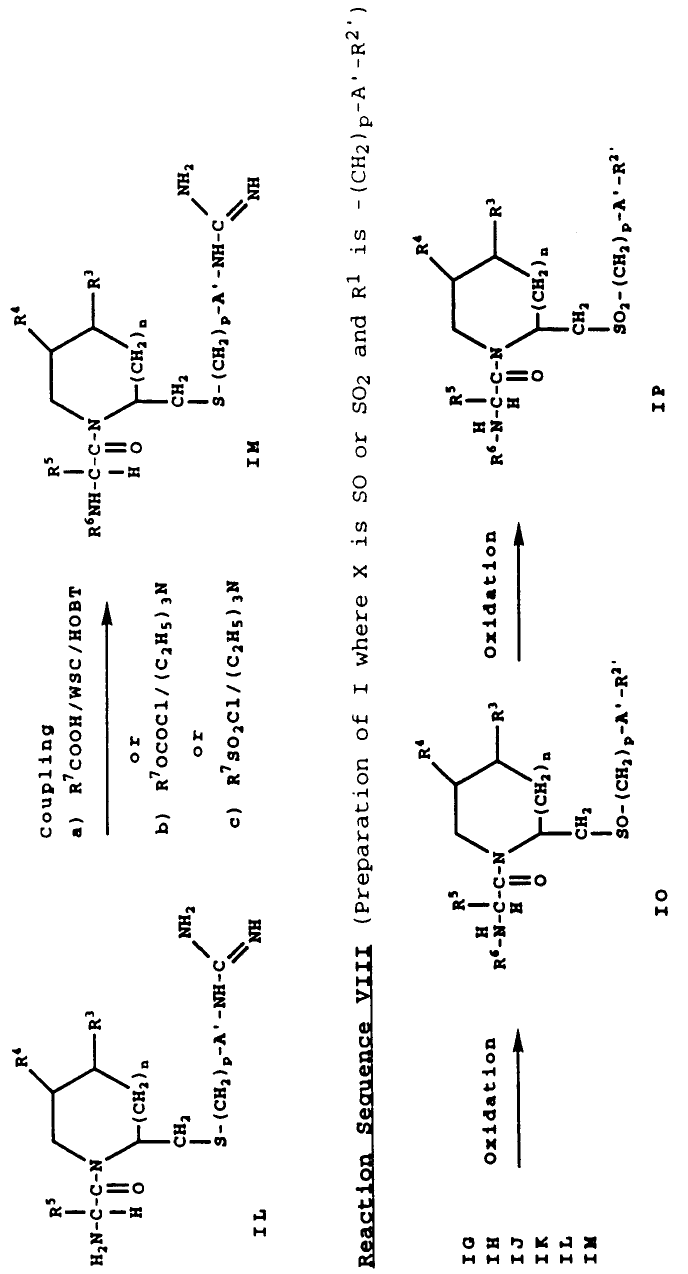

### Reaction Sequence IX (Preparation of I where R¹ is -A'R^{2'})

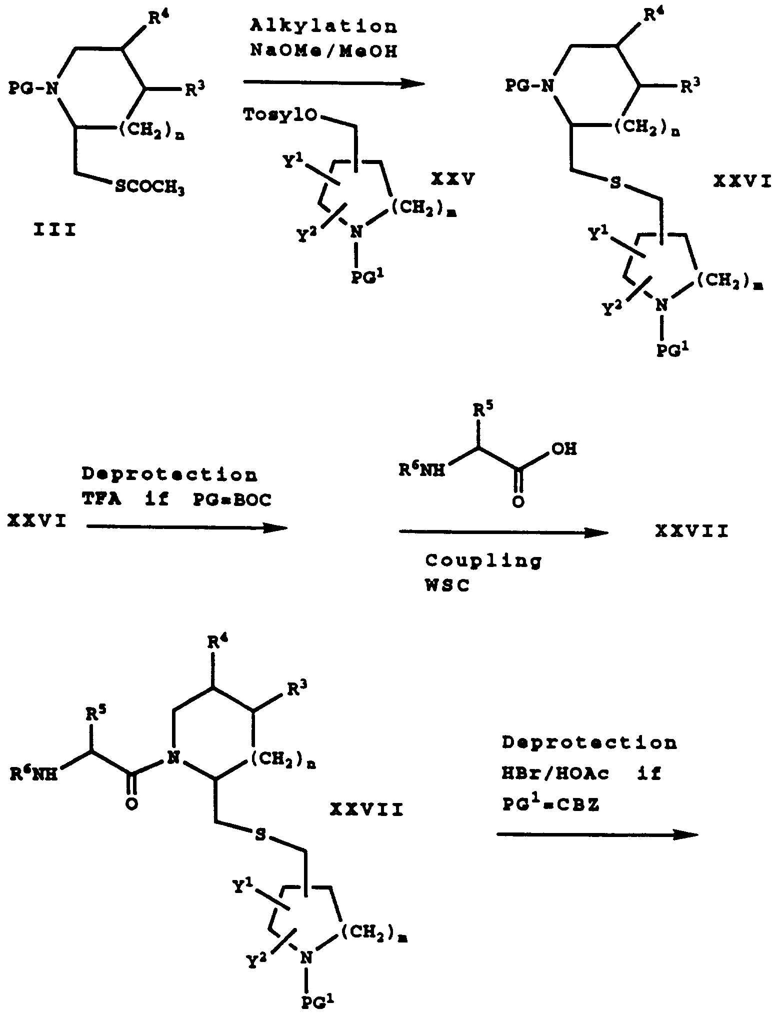

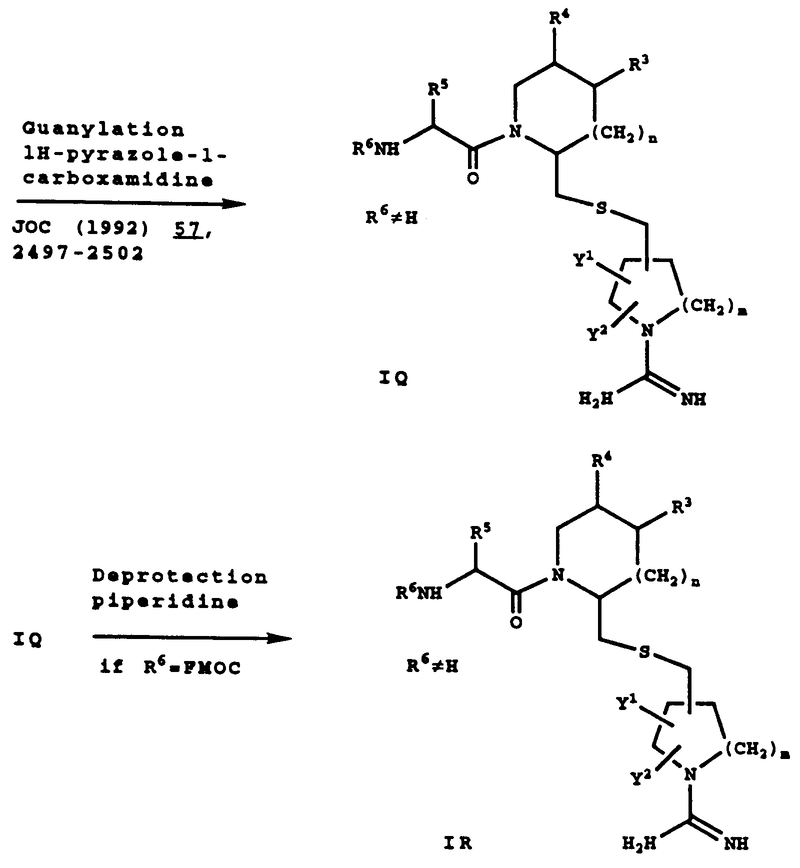

### Reaction Sequence X (Preparation of I where X is oxygen)

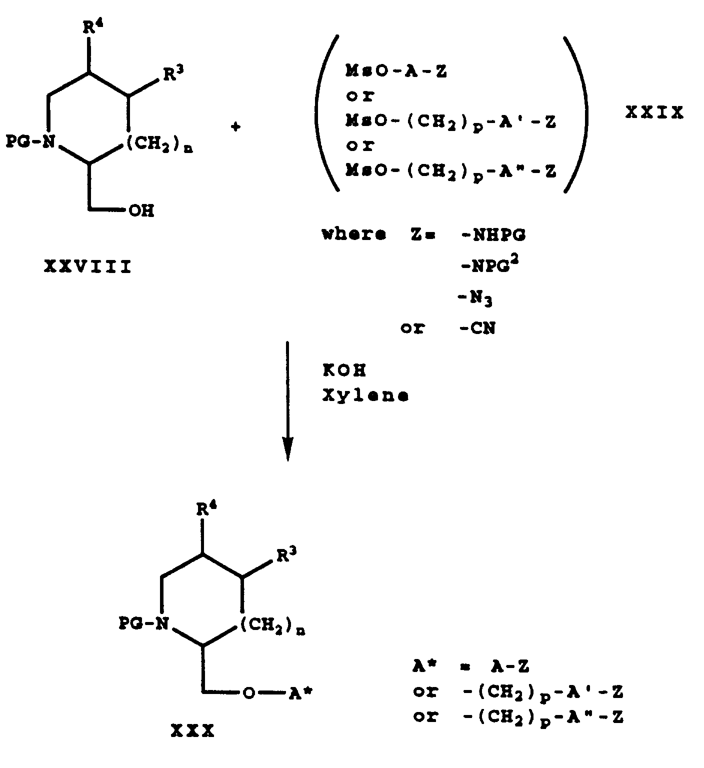

Referring to Reaction Sequence I compounds of formula I wherein X is S and R¹ is -A-R² and R² is amino may be prepared starting with tosylate II (prepared as described in J. Med. Chem. 35, 26l5 (l992)) (wherein PG in II is a protecting group such as 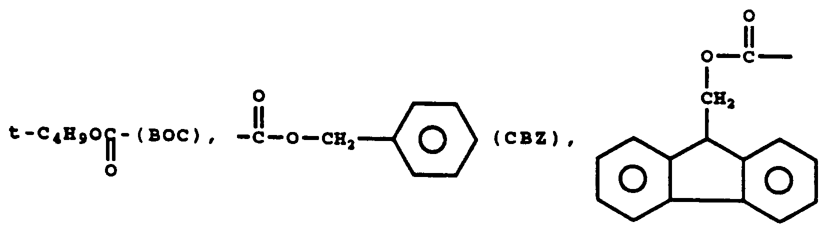
(fluorenylmethoxycarbonyl, FMOC), preferably BOC, which is subjected to a displacement reaction wherein II is treated with potassium thioacetate (KSAc) in the presence of an inert organic solvent such as acetone, dimethylformamide (DMF) or tetrahydrofuran (THF), under an inert atmosphere such as argon, at a temperature within the range of from about 0° to about 100°C to form thioacetate III. Thioacetate III is then alkylated by reacting III with an alkali metal alkoxide such as sodium methoxide or potassium t-butoxide in an inert organic solvent such as THF, DMF or diethylether, in the presence of an alcohol solvent such as methanol or ethanol under an inert atmosphere such as argon, at a temperature within the range of from about -30° to about 50°C. To the resulting solution is added N-bromoalkyl-phthalimide A to form the thiophthalimide IV. Thiophthalimide IV is deprotected by treatment with a deprotecting agent such as trifluoroacetic acid where P.G. is BOC or HBr/acetic acid where P.G. is CBZ, with or without the presence of dry inert organic solvent such as dichloromethane, chloroform or THF, to form a crude amine salt IVA. 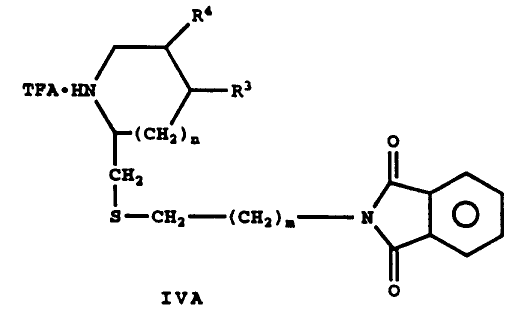
The resulting salt IVA is made to undergo a carbodiimide coupling reaction with protected amino acid B 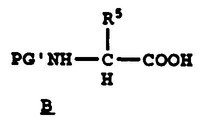
(wherein PG' is a protecting group which may be any of the PG protecting groups) in the presence of l-hydroxybenzotriazole monohydrate (HOBT), ethyl-3-(3-dimethylamino)propyl carbodiimide (WSC) or dicyclohexylcarbodiimide (DCC), and N-methylmorpholine (NMM), in the presence of an inert organic solvent such as dimethylformamide (DMF), THF or N-methylpyrrolidone, to form carbamate V.

Carbamate V is deprotected by treatment with trifluoroacetic acid (TFA) or other deprotecting agent such as HBr/HOAc (depending on P.G.'), with or without the presence of dry inert organic solvent such as dichloromethane, chloroform or THF, to give a crude trifluoroacetic acid amine salt VI (where the deprotecting agent is TFA).

The crude amine salt VI is then subjected to a coupling reaction wherein it is reacted with
a) R⁷COOH in the presence of WSC or DCC, HOBT and NMM,
b) R⁷SO₂Cl in the presence of triethylamine or
c) R⁷OCOCl in the presence of triethylamine, to form VII.

VII is then reacted with anhydrous hydrazine in the presence of dry solvent such as dichloromethane, chloroform or THF, and an alcohol solvent such as methanol or ethanol, to form thioalkylamine compound IA of the invention.

Compounds of formula I of the invention where R⁶ is H, X is S and R¹ is -A-R² and R² is NH₂ may be prepared as shown in Reaction Sequence IB) where amine VI is reacted with anhydrous hydrazine (as described above in the reaction of VII) to form IA'.

Compounds of formula I of the invention wherein X is S, R¹ is -A-guanidinyl and R⁶ is not H may be prepared as shown in Reaction Sequence IIA) wherein IA is subjected to a guanylation by reacting IA with amidine sulfonic acid or other guanylating agent such as lH-pyrazole-l-carboxamidine, in the presence of a weak organic base such as triethylamine, pyridine or N-methylmorpholine (NMM) and an alcohol solvent such as ethanol or methanol, to form IB.

Compounds of formula I of the invention wherein X is S, R¹ is -A-guanidinyl and R⁶ is H may be prepared as outlined in Reaction Sequence IIB wherein compound V is treated with hydrazine (as described above in Sequence IA in the reaction of VI) to form amine VA which is subjected to a guanylation reaction (as described above in Sequence IIA) to form protected guanidine VB which is deprotected by treatment with TFA where P.G.' is BOC or using other deprotecting agent as described hereinbefore, to form compound of the invention IB'.

Referring to Reaction Sequence IIIA, compounds of formula I of the invention wherein X is S and R¹ is -A-amidinyl and R⁶=H are prepared by alkylating III by treating III with an alkali metal alkoxide and with nitrile (C) in the presence of methanol or dimethylformamide, at a temperature within the range of from about -60° to about 75°C, to form nitrile VIII which is made to undergo amidine formation by treating nitrile VIII with hydrochloric acid in the presence of an alcohol such as methanol or ethanol, and then with ammonia to form amidine compound IX. Compound IX is then subjected to a carbodiimide coupling reaction by treating IX with B in the presence of WSC and HOBT (as described above in the reaction of IV to form V in Sequence I), to form X which is deprotected as described hereinbefore to form IC'.

Compounds of formula I of the invention wherein X is S, R¹ is -A-amidinyl and R⁶ is not H, are prepared as shown in Sequence IIIB where IC' is treated with a coupling agent R⁷COOH, R⁷OCOCl or R⁷SO₂Cl (as described above in Sequence IA) in the coupling of VI to form VII), to thereby form IC.

Referring to Reaction Sequence IV, compounds of formula I wherein X is SO and R¹ is -A-R² and R² is NH₂, may be prepared by oxidizing amide VI or VII by treating VI or VII with an oxidizing agent such as m-chloroperbenzoic acid (MCPBA), oxone or sodium periodate in the presence of an inert organic solvent such as dichloromethane, chloroform or acetonitrile, employing from about 0.9 to about 1.5 moles of oxidizing agent per mole of VI or VII, to form the corresponding sulfoxide (X is SO). The corresponding sulfone (X is SO₂) is formed employing from about 2 to about 3 moles of oxidizing agent per mole of VI or VII. The so-formed sulfoxide or sulfone is then deprotected with hydrazine (as described in Sequence IA in the reaction of VI) to form the corresponding amine ID of the invention.

As seen in Sequence IV B1 where R⁶≠H and R² is guanidine, the resulting amine ID is guanylated by reaction with amidine sulfonic acid (as described hereinbefore in forming compounds of formula I where X is S) to form compounds of formula I where X is SO or SO₂ (that is IE).

Where R⁶ is H and R¹ is -A-guanidinyl (Sequence IVB2) compounds of formula I of the invention wherein X is SO or SO₂ are formed as follows: compound V is oxidized as described above for VI or VII, to form compound XII which is then reacted with hydrazine to form XIII which is guanylated and then deprotected to form compound of the invention IE' employing procedures described hereinbefore.

Sulfoxides or sulfones of compounds of formula I of the invention where R¹ is -A-amidinyl are prepared as shown in Sequence IVC) by oxidizing IC or IC' employing procedures as described above to form compound of the invention IF.

Referring to Reaction Sequence V, compounds of formula I wherein X is S and R¹ is -A'R^{2'} and R^{2'} is amidinyl, may be prepared starting with tosylate II which is subjected to a displacement reaction wherein II is treated with nitrile D

D HS-A'-CN

in the presence of a base such as sodium methoxide, triethylamine or pyridine, to form nitrile XV. Nitrile XV is admixed with strong acid such as hydrochloric acid, and an alcohol solvent such as methanol, or ethanol and made to undergo amidine formation by treatment with ammonia to form the corresponding amidine XVI. The resulting amidine compound XVI is then subjected to a carbodiimide coupling reaction with protected amino acid B in the presence of WSC or DCC, and HOBT and NMM, as described with respect to Sequence I, to form amide XVII. Amide XVII is deprotected by treatment with trifluoroacetic acid or other deprotecting agent, as described with respect to Sequence I, to form compound IG of the invention, which may be subjected to a coupling reaction wherein it is reacted with R⁷COOH, R⁷SO₂Cl or R⁷OCOCl, as described with respect to Sequence I, to form compounds of formula IH wherein X is S and R¹ is -A'R^{2'} and R^{2'} is amidinyl.

Referring to Reaction Sequence VI, compounds of formula I of the invention wherein X is S, R¹ is -A'-R^{2'} and R^{2'} is aminomethyl, may be prepared by reducing compound XV, for example using a reducing agent such as lithium aluminum hydride, triethylborohydride or diborane, to form amine XVIII which is treated with a protecting agent E such as CBZ-Cl to form XIX. Protected amine XIX is then treated with HCl in the presence of an alcohol such as methanol or dioxane and then treated with coupling agent (B), as described hereinbefore with respect to Sequence I, to form compound XX. Compound XX may be completely deprotected with e.g., HBr/HOAc to give amine IJ. Alternatively, XX may be selectively deprotected with e.g., TFA (if PG'=BOC) and then subjected to a coupling reaction by treatment with coupling agent R⁷COOH, R⁷OCOCl or R⁷SO₂Cl, followed by removal of PG² with HBr/HOAc, to form amine IK of the invention.

Referring to Reaction Sequence VII, compounds of formula I wherein X is S, R¹ is -A'-R^{2'} and R^{2'} is guanidinyl may be prepared by guanylating amine XXI to form guanidine XXII which is reacted with II to form guanidine XXIII. Guanidine XXIII is deprotected and then made to undergo a coupling reaction with B, as described with respect to Sequence I, to form XXIV which is deprotected to form guanidine IL of the invention. Guanidine IL may then be reacted with a coupling agent R⁷COOH, R⁷OCOCl or R⁷SO₂Cl, as described with respect to Sequence I, to form guanidine IM of the invention.

Referring to Reaction Sequence VIII, compounds of formula I of the invention wherein R¹ is -A'-R^{2'} and X is SO may be prepared by oxidizing IG, IH, IJ, IK, IL or IM by treating same with an oxidizing agent such as m-chloroperbenzoic acid (MCPBA), oxone or sodium periodate in the presence of an inert organic solvent such as dichloromethane, employing from about 0.9 to about l.5 moles of oxidizing agent per mole of amide, to form the corresponding sulfoxide. The corresponding sulfone may be formed employing from about 2 to about 3 moles of oxidizing agent per mole of amide. The so-formed sulfoxide or sulfone is then deprotected with hydrazine and the resulting amine is guanylated by reaction with amidine sulfonic acid (as described hereinbefore in forming compounds of formula I where X is S) to form compounds of formula I where X is SO or SO₂.

As seen in reaction sequence IX, compounds of formula IQ and IR may be prepared by treating thioacetate III with sodium methoxide, followed by an alkylating agent of formula XXV to provide a compound of formula XXVI. Deprotection of XXVI (by TFA if PG=BOC) and carbodiimide coupling, as described previously, affords compound XXVII. Removal of the protecting group PG' (by HBr/HOAc if PG' = CBZ) followed by guanylation using 1H-pyrazole-1-carboxamidine (JOC 1992, *57*, 2497-2502) then provides a compound of formula IQ where R⁶ is not H. The compound IQ can be deprotected by piperidine (if R⁶ = fluorenylmethyloxycarbonyl (FMOC)) to obtain compounds IR where R⁶ = H.

The key step for the preparation of compounds wherein X is oxygen is shown in Scheme X. Treatment of the protected alcohol XXVIII with KOH and a mesylate (Ms) of formula XXIX in xylene (see J. Med. Chem 1986, *29*, 2335-2347) will provide the series of compounds XXX. Further steps in the preparation of compounds of formula I wherein X is oxygen may be effected in analogy with the previously described Schemes I, II, III, V, VI, VII, and IX.

The compounds of formula I of the invention can be obtained as pharmaceutically acceptable acid addition salts by reacting a free base with an acid, such as hydrochloric, hydrobromic, hydroiodic, nitric, sulfuric, phosphoric, acetic, citric, maleic, succinic, lactic, tartaric, gluconic, benzoic, methanesulfonic, ethanesulfonic, benzenesulfonic, p-toluenesulfonic acid or the like.

The compounds of the present invention are serine protease inhibitors, and in particular may inhibit thrombin, Factor Xa, and/or trypsin. The compounds of the present invention are useful for the treatment or prophylaxis of those processes which involve the production and/or action of thrombin. This includes a number of thrombotic and prothrombotic states in which the coagulation cascade is activated which include, but are not limited to, deep vein thrombosis (DVT), disseminated intravascular coagulopathy (DIC), Kasabach-Merritt syndrome, pulmonary embolism, myocardial infarction, stroke, thromboembolic complications of surgery (such as hip replacement and endarterectomy) and peripheral arterial occlusion. In addition to its effects on the coagulation process, thrombin has been shown to activate a large number of cells (such as neutrophils, fibroblasts, endothelial cells, smooth muscle cells). Therefore, the compounds of the present invention may also be useful for the treatment or prophylaxis of adult respiratory distress syndrome, septic shock, septicemia, inflammatory responses which include, but are not limited to, edema, acute or chronic atherosclerosis, and reperfusion damage.

The compounds of the invention may also be useful in treating neoplasia/metastasis (in particular those which utilize fibrin) and neurodegenerative diseases such as Alzheimer's disease and Parkinson's disease. In addition, the compounds of the present invention may be useful to prevent restenosis following arterial injury induced by endogenous (rupture of an atherosclerotic plaque) or exogenous (invasive cardiological procedure) events.

The compounds of the present invention may also be used as an anticoagulant in extracorpeal blood circuits, such as those necessary in dialysis and surgery (such as coronary artery bypass surgery).

The compounds of the present invention may also be used in combination with thrombolytic agents, such as tissue plasminogen activator (natural or recombinant), streptokinse, urokinase, prourokinase, anisolated streptokinase plasminogen activator complex (ASPAC), animal salivary gland plasminogen activators, and the like. The compounds of the present invention may act in a synergistic fashion to prevent reocclusion following a successful thrombolytic therapy and/or reduce the time to reperfusion. The compounds of the present invention may also allow for reduced doses of the thrombolytic agent to be used and therefore minimize potential hemorrhagic side-effects.

The compounds of the present invention may also be used in combination with other antithrombotic or anticoagulant drugs such as thromboxane receptor antagonists, prostacyclin mimetics, phosphodiesterase inhibitors, fibrinogen antagonists, and the like.

Compounds of the present invention that inhibit trypsin may also be useful for the treatment of pancreatitis.

The compounds of the invention can be administered orally or parenterally to various mammalian species known to be subject to such maladies, e.g., humans, cats, dogs and the like in an effective amount within the dosage range of about 0.l to about l00 mg/kg, preferably about 0.2 to about 50 mg/kg and more preferably about 0.5 to about 25 mg/kg (or from about l to about 2500 mg, preferably from about 5 to about 2000 mg) on a regimen in single or 2 to 4 divided daily doses.

The active substance can be utilized in a composition such as tablet, capsule, solution or suspension containing about 5 to about 500 mg per unit of dosage of a compound or mixture of compounds of formula I. They may be compounded in conventional matter with a physiologically acceptable vehicle or carrier, excipient, binder, preservative, stabilizer, flavor, etc., as called for by accepted pharmaceutical practice.

The following Examples represent preferred embodiments of the present invention. Unless otherwise indicated, all temperatures are expressed in degrees Centigrade.

### Example l

### [S-(R*,R*)]-N-[2-[2-[[[4-[(Aminoiminomethyl)amino]-butyl]thio]methyl]-l-pyrrolidinyl]-l-(hydroxymethyl)2-oxoethyl]-2-naphthalenesulfonamide, trifluoroacetate (l:l) salt

### A. (S)-2-[[[4-(l,3-Dihydro-l,3-dioxo-2H-isoindol-2-yl)butyl]thio]methyl]-l-pyrrolidinecarboxylic acid, l,l-dimethylethyl ester

To a stirred solution of (S)-2-[[[(4-methylphenyl)sulfonyl]oxy]methyl]-l-pyrrolidinecarboxylic acid, l,l-dimethylethyl ester (reported in Journal of Med. Chemistry, p2615, 1992) (10.0 g, 28.2 mmol) in 100 mL of acetone under argon was added potassium thioacetate (3.91 g, 34.2 mmol). The reaction solution was refluxed for 18 h at which time another batch of potassium thioacetate (3.22 g, 28.2 mmol) was added. This mixture was refluxed for 24 h and cooled to room temperature. The precipitate was filtered off through a 2" pad of Celite and rinsed with acetone (2x30 mL). The filtrate was concentrated *in vacuo*. The residue was dissolved in 300 mL of ether and washed with water (2x50 mL) and brine (1x50 mL). The organic layer was dried (Na₂SO₄), filtered and concentrated *in vacuo* to give 6.75 g of the intermediate thioacetate. To a stirred solution of this thioacetate (3.27 g, 12.6 mmol) in 100 mL of methanol under argon at 0°C was added a solution of 1M t-C₄H₉OK in tetrahydrofuran (THF) (12.6 mL, 12.6 mmol). This reaction solution was then sparged with argon for 15 min. To this solution was added N-(4-bromobutyl)phthalimide (3.56 g, 12.6 mmol). The reaction solution was stirred at room temperature for 3 h and quenched at 0°C by dropwise addition of 20 mL of saturated NH₄Cl solution. The mixture was concentrated *in vacuo*. The residue was diluted with 600 mL of EtOAc and washed with saturated NaHCO₃ solution (2x200 mL) and brine (1x200 mL). The EtOAc layer was dried (MgSO₄), filtered and concentrated *in vacuo*. Purification was effected by flash chromatography on 180 g of Merck silica gel 60 using 29% ether in hexane as eluant to give 3.16 g (55%) of title thioether.

### B. [S-(R*,R*)]-[2-[2-[[[4-(l,3-Dihydro-l,3-dioxo-2H-isoindol-2-yl)butyl]thio]methyl]-l-pyrrolidinyl]-l-(hydroxymethyl)-2-oxoethyl]-carbamic acid, l,l-dimethylethyl ester

To a stirred solution of Part A thioether (3.28 g, 7.85 mmol) in 10 mL of dry dichloromethane was added 10 mL of trifluoroacetic acid (TFA). The solution was stirred at room temperature for 1.5 h and diluted with 100 mL of ether. The solution was concentrated *in vacuo* to give a crude TFA·amine salt. To a stirred solution of this salt, 1-hydroxybenzotriazole monohydrate (HOBT) (1.33 g, 7.85 mmol) and N-Boc-L-serine (1.70 g, 7.85 mmol) in 40 mL of dimethylformamide (DMF) was added in order N-methylmorpholine (NMM) (2.58 mL, 23.5 mmol) and ethyl-3-(3-dimethylamino)propyl carbodiimide hydrochloride (1.50 g, 7.85 mmol). This reaction solution was sparged with argon for 10 min and stirred at room temperature for 18 h. The mixture was concentrated under pump vacuum at 45°C. The residue was dissolved in 200 mL of EtOAc and washed with 1N HCl solution (2x80 mL), saturated NaHCO₃ solution (1x80 mL) and brine (1x80 mL). The EtOAc layer was dried (MgSO₄), filtered and concentrated *in vacuo* to give 3.65 g (90%) of title carbamate.

### C. [S-(R*,R*)]-N-[2-[2-[[[4-(l,3-Dihydro-l,3-dioxo-2H-isoindol-2-yl)butyl]thio]methyl]-l-pyrrolidinyl]-l-(hydroxymethyl)-2-oxoethyl]-2-naphthalenesulfonamide

To a stirred solution of Part B carbamate (3.65 g, 7.06 mmol) in 15 mL of dry dichloromethane was added 15 mL of TFA. The solution was stirred at room temperature for 1.5 h and diluted with 200 mL of ether. The solution was concentrated *in vacuo* to give a crude TFA·amine salt. To a stirred solution of this salt and triethyl amine (2.95 mL, 21.2 mmol) in 60 mL of dry dichloromethane at 0°C was added 2-naphthalenesulfonyl chloride (1.76 g, 7.77 mmol). The reaction solution was stirred at room temperature for 4.5 h and diluted with 140 mL of dichloromethane. The solution was washed with 1N HCl solution (2x60 mL), saturated NaHCO₃ solution (2x60 mL) and brine (1x60 mL). The organic layer was dried (MgSO₄), filtered and concentrated *in vacuo*. This was chromatographed on 120 g of Merck silica gel 60 using 2% CH₃OH/CH₂Cl₂ as eluant to give 2.62 g (61%) of title sulfonamide.

### D. [S-(R*,R*)]-N-[2-[2-[[(4-Aminobutyl)thio]methyl]-l-pyrrolidinyl]-l-(hydroxymethyl)-2-oxoethyl]-2-naphthalenesulfonamide

To a stirred solution of Part C sulfonamide (0.77 g, 1.27 mmol) in 20 mL of dry dichloromethane under argon was added anhydrous hydrazine (0.24 g, 7.61 mmol). The reaction mixture was stirred at room temperature for 2.5 h and concentrated *in vacuo*. The residue was combined with 30 mL of methanol and 60 mL of toluene. The mixture was concentrated *in vacuo*. This co-evaporation with methanol and toluene was repeated three times. The residue was dissolved in 80 mL of methanol and the solution was heated to reflux for 17 h. The mixture was then cooled to room temperature and acidified to pH 1 by the addition of 1N HCl in ether. The mixture was cooled in an acetone dry ice bath for 5 min and the precipitate was filtered off, the solid was rinsed with methanol (2x10 mL), and the filtrate was concentrated *in vacuo*. The product was purified by prep HPLC and lyophilized to give 290 mg (39%) of title amine·TFA.

### E. [S-(R*,R*)]-N-[2-[2-[[[4-[(Aminoiminomethyl)amino]butyl]thio]methyl]-l-pyrrolidinyl]-l-(hydroxymethyl)-2-oxoethyl]-2-naphthalenesulfonamide, trifluoroacetate (l:l) salt

To a stirred mixture of Part D amine·TFA (280 mg, 0.47 mmol) and Et₃N (0.36 mL, 2.61 mmol) in 4 mL of absolute EtOH under argon was added aminoiminomethanesulfonic acid (206 mg, 1.66 mmol). The mixture was stirred at room temperature for 3 h and concentrated *in vacuo*. This was purified by prep HPLC to give 180 mg (57%) of title compound.
Opt. Rotation: [α]_{D}= -54.7° (c=0.70, MeOH).

| Analysis calc'd for 0.33 H₂O + l.35 TFA: | | | | | |
|---|---|---|---|---|---|
| | C, 46.24; | H, 5.29; | N, l0.49; | S, 9.6l; | F, ll.53 |
| Found: | C, 46.24; | H, 5.22; | N, l0.47; | S, 9.92; | F, ll.47. |

### Example 2

### [S-(R*,R*)]-N-[4-[[[l-(2-Amino-l-oxo-3-phenylpropyl)-2-pyrrolidinyl]methyl]thio]butyl]guanidine, trifluoroacetate (2:5) salt

### A. [S-(R*,S*)]-[2-[2-[[[4-(l,3-Dihydro-l,3-dioxo-2H-isoindol-2-yl)butyl]thio]methyl]-l-pyrrolidinyl]-2-oxo-l-(phenylmethyl)ethyl]carbamic acid, l,l-dimethylethyl ester

To a stirred solution of TFA·amine (prepared in Example l, Part B) (3.11g, 7.20 mmol), 1-hydroxybenzotriazole monohydrate (1.22 g, 7.20 mmol) and N-Boc-D-phenylalanine (1.91 g, 7.20 mmol) in 60 mL of DMF was added in order 4-methylmorpholine (3.16 mL, 28.8 mmol) and ethyl-3-(3-dimethylamino)propyl carbodiimide hydrochloride (1.38 g, 7.20 mmol). This reaction solution was sparged with argon for 20 min and stirred at room temperature for 18 h. The mixture was concentrated under pump vacuum at 45°C. The residue was dissolved in 400 mL of EtOAc and washed with 1N HCl solution (2x100 mL), saturated NaHCO₃ solution (2x100 mL) and brine (1x100 mL). The EtOAc layer was dried (MgSO₄), filtered and concentrated *in vacuo* . Purification was effected by flash chromatography to give 2.74 g (68%) of title carbamate.

### B. [S-(R*,S*)]-[2-[2-[[(4-Aminobutyl)thio]methyl]-l-pyrrolidinyl]-2-oxo-l-(phenylmethyl)ethyl]carbamic acid, l,l-dimethylethyl ester

To a stirred solution of Part A carbamate (1.01 g , 1.79 mmol) in 25 mL of methanol under argon was added anhydrous hydrazine (1.00 mL, 31.9 mmol). The reaction solution was heated at 50°C for 3 h and concentrated *in vacuo*. The residue was combined with 50 mL, of methanol and 20 mL of toluene. The mixture was concentrated *in vacuo*. The residue was diluted with 40 mL of 1N NaOH solution and extracted with EtOAc (3x70 mL). The combined organic extracts were dried (MgSO₄), filtered and concentrated *in vacuo* to give 700 mg (89%) of title amine.

### C. [S-(R*,S*)]-[2-[2-[[[4-[(Aminoiminomethyl)-amino]butyl]thio]methyl]-1-pyrrolidinyl]-2-oxo-l-(phenylmethyl)ethyl]carbamic acid, l,l-dimethylethyl ester

To a stirred mixture of Part B amine (680 mg, 1.56 mmol) and triethylamine (Et₃N) (0.76 mL, 5.47 mmol) in 7 mL of absolute EtOH under argon was added aminoimino-methanesulfonic acid (485 mg, 3.91 mmol). The mixture was stirred at room temperature for 2 h concentrated *in vacuo*, and purified by prep HPLC to give 780 mg (92%) of title BOC·amine.

### D. [S-(R*,R*)]-N-[4-[[[l-(2-Amino-l-oxo-3-phenylpropyl)-2-pyrrolidinyl]methyl]thio]butyl]guanidine, trifluoroacetate (2:5) salt

To Part C BOC·amine (410 mg, 0.69 mmol) at 0°C was added 4 ml, of TFA. The reaction solution was stirred at room temperature for 30 min and diluted with 20 mL of methanol. The solution was concentrated *in vacuo* and purified by prep HPLC to give 130 mg (28%) of title aminoguanidine.
Opt. rotation: [α]_{D}= -73.3° (c=0.54, MeOH).

| Analysis calc'd for l.03 H₂O + 2.30 TFA: | | | | | |
|---|---|---|---|---|---|
| | C, 43.06; | H, 5.4l; | N, l0.64; | S, 4.87; | F, l9.9l |
| Found: | C, 43.06; | H, 5.l4; | N, l0.40; | S, 5.22; | F, 20.00. |

### Example 3

### [S-(R*,R*)]-N-[2-[2-[[[4-[(Aminoiminomethyl)amino]butyl]sulfonyl]methyl]-l-pyrrolidinyl]-l-(hydroxymethyl)-2-oxoethyl]-2-naphthalenesulfonamide, trifluoroacetate (l:l) salt

### A. [S-(R*,R*)]-N-[2-[2-[[[4-(l,3-Dihydro-l,3-dioxo-2H-isoindol-2-yl)butyl]sulfonyl]methyl]-l-pyrrolidinyl]-l-(hydroxymethyl)-2-oxoethyl]-2-naphthalenesulfonamide

To a stirred solution of the thioether prepared in Example l, Part C (1.00 g, 1.65 mmol) in 50 mL of dichloromethane was added m-chloroperbenzoic acid (MCPBA) (1.14 g, 3.29 mmol). The reaction solution was stirred at room temperature for 10 min and quenched by the addition of 10% sodium bisulfite solution until the KI-starch paper test was negative. The mixture was diluted with 300 mL of EtOAc and washed with saturated NaHCO₃ solution (2x150 mL) and brine (1x150 mL). The organic layer was dried (MgSO₄), filtered and concentrated *in vacuo* to give 1.20 g (97%) of title sulfone.

### B. [S-(R*,R*)]-N-[2,[2-[[(4-Aminobutyl)sulfonyl]methyl]-l-pyrrolidinyl]-l-(hydroxymethyl)-2-oxoethyl]-2-naphthalenesulfonamide

To a stirred solution of Part A sulfone (1.20 g , 1.96 mmol) in 30 mL of methanol under argon was added anhydrous hydrazine (0.63 g, 19.6 mmol). The reaction solution was heated to reflux for 2.5 h and concentrated *in vacuo*. The residue was combined with 40 mL of methanol and 80 mL of toluene. The mixture was concentrated *in vacuo*, the residue was diluted with 40 mL of 1N NaOH solution and extracted with EtOAc (2x80 mL) and 10% isopropyl alcohol (IPA) in dichloromethane (3x80 mL). The combined organic extracts were dried (MgSO₄), filtered, concentrated *in vacuo* and purified by prep HPLC to give 350 mg (30%) of title amine·TFA.

### C. [S-(R*,R*)]-N-[2-[2-[[[4-[(Aminoiminomethyl)amino]butyl]sulfonyl]methyl]-l-pyrrolidinyl]-l-(hydroxymethyl)-2-oxoethyl]-2-naphthalenesulfonamide, trifluoroacetate (l:l) salt

To a stirred mixture of Part B amine·TFA (340 mg, 0.57 mmol) and Et₃N (0.32 mL, 2.29 mmol) in 6 mL of absolute EtOH under argon was added aminoiminomethanesulfonic acid (177 mg, 1.43 mmol). The mixture was stirred at room temperature for 4 h concentrated *in vacuo*, and purified by prep HPLC to give 370 mg (92%) of title compound.
Opt. rotation: [α]_{D}= -53.3° (c=0.95, MeOH).

| Analysis calc'd for 0.l6 H₂O + l.40 TFA: | | | | | |
|---|---|---|---|---|---|
| | C, 44.l3; | H, 4.98; | N, 9.97; | S, 9.l3; | F, ll.36 |
| Found: | C, 44.l3; | H, 4.87; | N, 9.97; | S, 9.05; | F, ll.48. |

### Example 4

### [S-(R*,R*)]-N-[2-[2-[[[3-[(Aminoiminomethyl)amino]propyl]thio]methyl]-l-pyrrolidinyl]-l-(hydroxymethyl)-2-oxoethyl]-2-naphthalenesulfonamide, trifluoroacetate (l:l) salt

### A. (S)-2-[[[3-(l,3-Dihydro-l,3-dioxo-2H-isoindol-2-yl)propyl]thio]methyl]-l-pyrrolidinecarboxylic acid, l,l-dimethylethyl ester

To a stirred solution of the tosylate employed as a starting material in Example l, Part A (10.0 g, 28.2 mmol) in 120 mL of acetone under argon was added potassium thioacetate (4.14 g, 36.2 mmol). The reaction solution was sparged with argon for 10 min. This mixture was refluxed for 4 h and cooled to room temperature. The precipitate was filtered off through a 3" pad of Celite and rinsed with acetone (3x30 mL). The filtrate was concentrated *in vacuo*. The residue was dissolved in 400 mL of ether and washed with cold water (1x100 mL) and brine (1x100 mL). The organic layer was dried (Na₂SO₄), filtered and concentrated *in vacuo* to give 7.20 g (99%) of the intermediate thioacetate. To a stirred solution of this thioacetate (7.20 g, 27.8 mmol) in 250 mL of methanol under argon at 0°C was added a solution of 1M t-BuOK in THF (27.8 mL, 27.8 mmol). This reaction solution was then sparged with argon for 15 min. To this solution was added N-(3-bromopropyl)-phthalimide (7.45 g, 27.8 mmol). The reaction solution was stirred at room temperature for 4 h and quenched at 0°C by dropwise addition of 40 mL of saturated NH₄Cl solution. The mixture was concentrated *in vacuo*, the residue diluted with 400 mL of EtOAc and washed with saturated NaHCO₃ solution (2x100 mL) and brine (1x100 mL). The EtOAc layer was dried (MgSO₄), filtered and concentrated *in vacuo*. Purification was effected by flash chromatography on to give 6.11 g (54%) of title thioether.

### B. [S-(R*,R*)]-[2-[2-[[[3-(l,3-Dihydro-l,3-dioxo-2H-isoindol-2-yl)propyl]thio]methyl]-l-pyrrolidinyl-l-(hydroxymethyl)-2-oxoethyl]carbamic acid, l,l-dimethylethyl ester

To a stirred solution of Part A thioether (6.00 g, 14.9 mmol) in 10 mL of dry dichloromethane was added 15 mL of TFA. The solution was stirred at room temperature for 1.5 h and diluted with 150 mL of ether. The solution was concentrated *in vacuo* to give a crude TFA·amine salt. To a stirred mixture of this TFA·amine salt in 100 mL of dry dichloromethane was added a solution of 1N HCl in ether (20.0 mL, 20.0 mmol). The mixture was concentrated *in vacuo* to give the amine·HCl salt in a quantitative yield. To a stirred solution of this HCl salt (4.05 g, 9.19 mmol), 1-hydroxybenzotriazole monohydrate (1.55 g, 9.19 mmol) and N-Boc-L-serine (2.00 g, 9.19 mmol) in 40 mL of DMF was added in order 4-methylmorpholine (4.04 mL, 36.8 mmol) and ethyl-3-(3-dimethylamino)propyl carbodiimide hydrochloride (1.76 g, 9.19 mmol). This reaction solution was sparged with argon for 10 min and stirred at room temperature for 22 h. The mixture was concentrated under pump vacuum at 45°C. The residue was dissolved in 300 mL of EtOAc and washed with 1N HCl solution (2x100 mL), saturated NaHCO₃ solution (1x100 mL) and brine (1x100 mL). The EtOAc layer was dried (MgSO₄), filtered and concentrated *in vacuo* to give 3.88 g (84%) of title carbamate.

### C. [S-(R*,R*)]-N-[2-[2-[[[3-(l,3-Dihydro-l,3-dioxo-2H-isoindol-2-yl)propyl]thio]methyl]-l-pyrrolidinyl]-l-(hydroxymethyl)-2-oxoethyl]-2-naphthalenesulfonamide

To a stirred solution of Part B carbamate (2.04 g, 4.06 mmol) in 10 mL of dry dichloromethane was added at 0°C a solution of 4N HCl in dioxane (15.0 mL, 60.0 mmol). The solution was stirred at room temperature for 1.5 h and diluted with 200 mL of ether. The solution was concentrated *in vacuo* to give a crude HCl·amine salt. To a stirred solution of this salt and triethyl amine (1.69 mL, 12.2 mmol) in 60 mL of dry dichloromethane at 0°C was added 2-naphthalenesulfonyl chloride (1.01 g, 4.46 mmol). The reaction solution was sparged with argon for 5 min and stirred at room temperature for 3 h. The reaction solution was diluted with 240 mL of dichloromethane. The solution was washed with 1N HCl solution (3x60 mL), saturated NaHCO₃ solution (2x60 mL) and brine (1x100 mL). The organic layer was dried (MgSO₄), filtered and concentrated *in vacuo*. The residue was washed with hexane (2x60 mL) and the solid was chromatographed on 120 g of Merck silica gel 60 using 3%CH₃OH/CH₂Cl₂ as eluant to give 1.69 g (70%) of title sulfonamide.

### D. [S-(R*,R*)]-N-[2-[2-[[[3-[(Aminoiminomethyl)amino]propyl]thio]methyl]-l-pyrrolidinyl]-l-(hydroxymethyl)-2-oxoethyl]-2-naphthalenesulfonamide, trifluoroacetate (l:l) salt

To a stirred solution of Part C sulfonamide (1.59 g, 1.68 mmol) in 40 mL of methanol under argon was added anhydrous hydrazine (0.54 g, 16.8 mmol). The reaction mixture was heated at 45°C for 5 h and concentrated *in vacuo*. The residue was combined with 60 mL of methanol and 100 mL of toluene. The mixture was concentrated *in vacuo* to give 1.07 g (85%) of the intermediate amine. To a stirred mixture of this amine (1.05 g, 2.25 mmol) and Et₃N (1.09 mL, 7.87 mmol) in 50 mL of absolute EtOH under argon was added aminoiminomethanesulfonic acid (698 mg, 5.62 mmol). The mixture was stirred at room temperature for 4 h, concentrated *in vacuo* and purified by prep HPLC to give 350 mg (25%) of title compound.
Opt. rotation: [α]_{D}= -63.l° (c=l.l2, MeOH).

| Analysis calc'd for 0.30 H₂O + l.07 TFA: | | | | | |
|---|---|---|---|---|---|
| | C, 46.69; | H, 5.30; | N, ll.28; | S, l0.32; | F, 9.82 |
| Found: | C, 46.64; | H, 5.24; | N, ll.l2; | S, l0.37; | F, 9.85. |

### Example 5

### [S-(R*,R*)]-N-[2-[2-[[[[l-(Aminoiminomethyl)-4-piperidinyl]methyl]thio]methyl]-l-pyrrolidinyl]-l-(hydroxymethyl)-2-oxoethyl]-2-naphthalenesulfonamide, trifluoroacetate (l:l) salt

### A. (S)-4-[[[[l-[(l,l-Dimethylethoxy)carbonyl]-2-pyrrolidinyl]methyl]thio]methyl]-l-piperidinecarboxylic acid, phenylmethyl ester

To a solution of the thioacetate intermediate prepared in Example l, Part A, 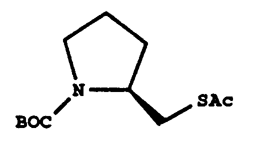
and 4-tosyloxymethyl-N-carbobenzyloxy piperidine, l.5 g, 3.86 mmol) in 35 mL DMF was added NaOMe (0.88 mL, 15.44 mmol) at 0°C. After the reaction stirred for l0 minutes and the ice bath was removed, the reaction was stirred at room temperature for 2 hrs. NaOMe (0.6 mL) was added and the reaction stirred for another 30 minutes. The reaction mixture was concentrated and partitioned between EtOAc and H₂O, the EtOAc layer washed with brine, dried over Na₂SO₄ and concentrated *in vacuo* to give the crude product which then was purified by a silica gel column (EtOAc:hexane=l:4) (87% yield).

### B. N-(2-Naphthalenylsulfonyl)-L-serine

To a solution of 2-naphthalene sulfonyl chloride (3 g, 13.23 mmol) and serine benzylester (3.07 g, l3.23 mmol) in l20 mL CH₂Cl₂ was added Et₃N (5.53 mL, l3.23 mmol) at 0°C. The reaction was stirred at room temperature for l hr and washed with a saturated (sat.) solution of KHSO₄, brine, and dried over Na₂SO₄. The organic layer was concentrated *in vacuo* to give the naphthylsulfonamide (93%).

To a solution of the naphthylsulfonamide (l g, 2.59 mmol) and Pd/C (l0%, 50 mg) in 25 mL EtOAc was added 2.6 mL of lN HCl, followed by H₂. The reaction was stirred at room temperature for l6 hrs and the catalyst was removed by filtration. The filtrate was concentrated *in vacuo* to give (89% yield).

### C. [S-(R*,R*)]-N-[l-(Hydroxymethyl)-2-oxo-2-[[[[[l-[(phenylmethoxy)carbonyl]-4-piperidinyl]methyl]thio]methyl]-l-pyrrolidinyl]ethyl]-2-naphthalenesulfonamide

To a solution of Part A compound (l.39 g, 3.l0 mmol) in 2 mL CH₂Cl₂ was added 9.3 mL trifluoroacetic acid (TFA) at 0°C. The ice bath was removed and the reaction was stirred at room temperature for 30 minutes. The reaction mixture was concentrated *in vacuo* and co-evaporated with Et₂O to give the TFA salt (70% yield).

To a solution of Part B compound (0.638 g, 2.l6 mmol) and HOBT (0.32l g, 2.37 mmol) in l0 mL, DMF was added WSC (0.46 g, 2.37 mmol) and the TFA salt (l00 g, 2.l6 mmol), followed by NMM (0.264 mL, 2.37 mmol). The reaction was stirred at room temperature for l6 hrs and partitioned between EtOAc and a sat. solution of NaHCO₃. The organic layer was further washed with a sat. solution of KHSO₄, brine, dried over Na₂SO₄ and concentrated *in vacuo* to give (8l% yield).

### D. [S-(R*,R*)]-N-[l-[(Acetyloxy)methyl]-2-oxo-2-[2-[[(4-piperidinylmethyl)thio]methyl]-l-pyrrolidinyl]ethyl]-2-naphthalenesulfonamide

To a solution of Part C compound (l.l g, 2.08 mmol) in l mL CH₂Cl₂ was added 6.24 mL HBr in HOAc (30%) at 0°C. The ice bath was removed and stirred at room temperature for l hr. The reaction mixture was concentrated *in vacuo* and triturated with Et₂O to give title compound.

### E. [S-(R*,R*)]-N-[l-[(Acetyloxy)methyl]-2-[2-[[[[l-(aminoiminomethyl)-4-piperidinyl]methyl]thio]methyl]-l-pyrrolidinyl]-2-oxoethyl]-2-naphthalenesulfonamide

To a solution of Part D (l.47 g, 2.57 mmol) and H-pyrazole-l-carboxamidine (0.42 g, 2.83 mmol) in 8 mL DMF was added diisopropylethylamine (0.898 mL, 5.l4 mmol). The reaction was stirred at room temperature for l6 hrs and purified by a preparative HPLC to give title compound (25% yield).

### F. [S-(R*,R*)]-N-[2-[2-[[[[l-(Aminoiminomethyl)-4-piperidinyl]methyl]thio]methyl]-l-pyrrolidinyl]-l-(hydroxymethyl)-2-oxoethyl]-2-naphthalenesulfonamide, trifluoroacetate (l:l) salt

To a solution of Part E compound (0.33 g, 0.57 mmol) in 2 mL MeOH was added lN NaOH (l.72 mL, l.72 mmol) at 0°C. The ice bath was removed and stirred at room temperature for l.5 hrs. The reaction mixture was concentrated *in vacuo* and purified on a preparative HPLC to give title compound (56% yield).

| Anal. Calc'd for C₂₅H₃₅N₅O₄S₂·l.2 TFA·l.2 H₂O: | | | | |
|---|---|---|---|---|
| | C, 47.55; | H, 5.62; | N, l0.l2; | S, 9.26 |
| Found: | C, 47.60; | H, 5.36; | N, 9.99; | S, l0.08. |

[α]_{D}= -l64.9 (c=3.04, CD₃OD).

### Example 6

### [S-(R*,R*)]-N-[2-[2-[[[l-(Aminoiminomethyl)-4-piperidinyl]methoxy]methyl]-l-pyrrolidinyl]-l-(hydroxymethyl)-2-oxoethyl]-2-naphthalenesulfonamide

### A. (S)-4-[[[l-[(l,l-Dimethylethoxy)carbonyl]-2-pyrrolidinyl]methoxy]methyl]-l-piperidinecarboxylic acid, phenylmethyl ester

To BOC-L-prolinol (10 mmol) in xylene (10 mL) was added KOH (20 mmol), 4-tosyloxymethyl-N-carbobenzyloxy piperidine (11 mmol) and the reaction was heated to reflux for 3 hours. The reaction mixture was stirred at room temperature for 16 hours, quenched with NH₄Cl, extracted with ethyl acetate and purified by column chromatography to provide the title ether.

### B. [S-(R*,R*)]-4-[[[l-[3-Hydroxy-2-[(2-naphthalenylsulfonyl)amino]-l-oxopropyl]-2-pyrrolidinyl]methoxy]methyl]-l-piperidinecarboxylic acid, phenylmethyl ester

The Part A ether (9 mmol) was dissolved in trifluoroacetic acid (10 mL) at 0°C. After stirring for 30 min, the TFA was evaporated in vacuo and the trifluoracetate salt precipitated with ether. The TFA salt was dissolved in DMF (10 mL) and reacted with N-2-napthylsulfonyl-serine (9 mmol) in the presence of WSC (9 mmol), HOBT (9 mmol) and NMM (9 mmol). After stirring for 12 hours, the reaction mixture was diluted with ethyl acetate (50 mL), washed with sat'd KHSO₄ (3X), sat'd NaHCO₃, sat'd NaCl, and dried in vacuo to provide the title naphthylsulfonyl serine derivative.

### C. [S-(R*,R*)]-N-[2-[2-[[[l-(Aminoiminomethyl)-4-piperidinyl]methoxy]methyl]-l-pyrrolidinyl]-l-(hydroxymethyl)-2-oxoethyl]-2-naphthalenesulfonamide

The Part B compound (8 mmol) was dissolved in HBr/HOAc at 0°C for 30 min. The reaction mixture was then made basic with NaOH and extracted with ethyl acetate. The free base derived from the ethyl acetate layer was treated with 1H-pyrazolecarboxamidine in ethanol at 40°C for 3 hours, the reaction mixture stripped and purified by preparative HPLC to provide the title compound.

### Example 7

### [S-(R*,S*)]-N-[2-[2-[[[4-[(Aminoiminomethyl)amino]butyl]thio]methyl]-l-pyrrolidinyl]-2-(hydroxymethyl)-2-oxoethyl]-2-naphthalenesulfonamide

The title compound was prepared employing a procedure similar to that described in Example l except substituting (R)-2-[[[(4-methylphenyl)sulfonyl]oxy]methyl]-l-pyrrolidine carboxylic acid, l,l-dimethylethyl ester for the corresponding (S)-isomer (l40 mg, 40% yield).

| Anal. Calc'd for C₂₃H₃₃N₅O₄S₂·l.l TFA·0.04 H₂O: | | | | | |
|---|---|---|---|---|---|
| | C, 47.8l, | H, 5.43; | N, ll.06; | S, l0.l3; | F, 9.90 |
| Found: | C, 47.75; | H, 5.35; | N, l0.93; | S, l0.l4; | F, 9.78 |

### Example 8

### [S-[R*,R*-(E)]]-N-[2-[2-[[(4-Amino-2-butenyl)thio]methyl]-l-pyrrolidinyl]-l-(hydroxymethyl)-2-oxo-ethyl]-2-naphthalenesulfonamide

### A. (S,Z)-2-[4-[(2-Pyrrolidinylmethyl)thio]-2-butenyl]-lH-isoindole-l,3(2H)-dione, hydrochloride

To a solution of (S,Z)-2-[4-[[[l-[(l,l-dimethylethoxy)carbonyl]-2-pyrrolidinyl]methyl]thio]-2-butenyl]-lH-isoindole-l,3(2H)-dione, (prepared as in Example l, Part A, except substituting 4-bromo-2-butenyl phthalimide for the corresponding phthalimide) (3.60 mmol)) in 2 mL of CH₂Cl₂ was added l8 mL of 4N HCl/dioxane at 0°C. The ice bath was removed and the reaction was stirred at room temperature for 3 hrs. The reaction mixture was concentrated *in vacuo* to give title compound (92% yield).

### B. [S-[R*,R*-(Z)]]-N-[2-[2-[[[4-(l,3-Dihydro-l,3-dioxo-2H-isoindol-2-yl)-2-butenyl]thio]methyl]-l-pyrrolidinyl]-2-(hydroxymethyl)-2-oxoethyl]-2-naphthalenesulfonamide

To a solution of Example 5, Part B comound (650 mg, 2.20 mmol) and HOBT (327 mg, 2.42 mmol) in l4 mL of DMF was added WSC (464 mg, 2.42 mmol), followed by the Part A amine salt (8l8 mg, 2.20 mmol). The pH was adjusted to 8 by NMM (0.27 mL, 2.42 mmol) and the reaction was stirred at room temperature for l6 hrs. The reaction mixture was partitioned between H₂O and EtOAc and EtOAc was washed with a sat. solution of NaHCO₃, a sat. solution of KHSO₄, brine and dried over MgSO₄. The organic layer was concentrated *in vacuo* to give title compound (86% yield).

### C. [S-[R*,R*-(E)]]-N-[2-[2-[[(4-Amino-2-butenyl)thio]-methyl]-l-pyrrolidinyl]-l-(hydroxymethyl)-2-oxoethyl]-2-naphthalenesulfonamide

To a solution of Part B compound (800 mg, l.34 mmol) in 34 mL CH₃OH was added hydrazine (0.42 mL, l3.4 mmol) at room temperature and the reaction was stirred at 40-50°C for 3 hrs. The reaction mixture was concentrated *in vacuo* and partitioned between EtOAc and lN NaOH; the aqueous layer was salted out with NaCl and extracted with EtOAc. The organic layers were combined and concentrated *in vacuo* to give the crude product which was crystallized in EtOAc (7l% yield).

| Anal. Calc'd for C₂₂H₂₉N₃O₄S₂·l.00 HCl·0.l4 H₂O: | | | | | |
|---|---|---|---|---|---|
| | C, 52.57; | H, 6.07; | N, 8.36; | S, l2.76; | Cl 7.05 |
| Found: | C, 52.84; | H, 6.0l; | N, 8.09; | S, l2.57; | Cl, 7.l4 |

[α]_{D}= -36.8 (c=0.50, MeOH).

### Example 9

### [S-[R*,R*-(E)]]-N-[2-[[[[4-[(Aminoiminomethyl)amino]-2-butenyl]thio]methyl]-l-pyrrolidinyl]-l-(hydroxymethyl)-2-oxoethyl]-2-naphthalenesulfonamide, trifluoroacetate (l:) salt

To a solution of Example 8 title compound (0.4 g, 0.86 mmol) and amidinesulfonic acid (l6l mg, l.29 mmol) in 5 mL EtOH was added Et₃N (0.364 mL, 2.59 mmol). The reaction was stirred at room temperature for l hr. The reaction mixture was concentrated *in vacuo* and purified by preparative HPLC to give title compound (60% yield).

| Anal. Calc'd for C₂₃H₃₁N₅O₄S₂·l.20 TFA·0.28 H₂O: | | | | | |
|---|---|---|---|---|---|
| | C, 47.ll; | H, 5.l0; | N, l0.82; | S, 9.90; | F, l0.56 |
| Found: | C, 47.l2; | H, 4.92; | N, l0.56; | S, 9.48; | F, l0.23 |

[α]_{D}= -26.6 (c=0.50, MeOH).

### Example l0

### [S-(R*,R*)]-N-[2-[2-[[[[l-(Aminoiminomethyl)-3-piperidinyl]methyl]thio]methyl]-l-pyrrolidinyl]-l-(hydroxymethyl)-2-oxoethyl]-2-naphthalenesulfonamide, trifluoroacetate (l:l) salt

The title compound was prepared in a manner similar to that described in Example 5, except substituting 3-tosyloxymethyl-N-carbobenzyloxy piperidine for 4-tosyloxymethyl-N-carbobenzyloxy piperidine, to provide the desired product.

| Anal. Calc'd for C₂₅H₃₅N₅O₄S₂·l.2 TFA·l.0l H₂O: | | | | |
|---|---|---|---|---|
| | C, 47.78; | H, 5.59; | N, l0.l7; | S, 9.3l |
| Found: | C, 47.78; | H, 5.46; | N, 9.97; | S, 9.37 |

[α]_{D}= -57.7 (c=2.0l, DMSO).

### Example ll

### [S-(R*,R*)]-N-[2-[2-[[[5-[(Aminoiminomethyl)amino]pentyl]thio]methyl]-l-pyrrolidinyl]-l-(hydroxymethyl)-2-oxoethyl]-l-naphthalenesulfonamide, trifluoroacetate (l:l) salt

The title compound was prepared as described in Example l, except N-(5-bromopentyl)phthalimide was substituted for the N-(4-bromobutyl)phthalimide.

| Anal. Calc'd for C₂₄H₃₅N₅O₄S·l.l6 TFA·0.92 H₂O: | | | | |
|---|---|---|---|---|
| | C, 47.l5; | H, 5.7l; | N, l0.44; | S, 9.56 |
| Found: | C, 47.l5; | H, 5.42; | N, l0.36; | S, 9.9l |

[α]_{D}= -263.7 (c=4.5l, CD₃OD).

### Example l2

### [S-(R*,R*)]-N-[2-[[2-[[[6-[(Aminoiminomethyl)amino]hexyl]thio]methyl]-l-pyrrolidinyl]-l-(hydroxymethyl)-2-oxoethyl]-2-naphthalenesulfonamide, trifluoroacetate (l:l) salt

### A. 6-Phthalimido-l-bromohexane

A solution of phthalic anhydride (32.0 g, 0.21 mol) and 6-aminohexanol (25.0 g, 0.21 mol) in 180 mL dry toluene was heated to reflux for 3 hrs. When the intermediate phthalimide was formed, PBr₃ (18.2 mL, 0.58 mol in 20 mL toluene) was added dropwise and the reaction was stirred at 100°C for 1.5 hrs. The reaction mixture was filtered thru a microfiber filter and the filtrate was concentrated in vacuo to give the crude product which then crystallized from toluene to give 6-phthalimido-1-bromohexane. (86% yield).

### B. N-Boc-2-pyrrolidinethio-6-hexaphthalimide

To a solution of the Example l Part A tosylate (8 g, 22.53 mmol) in l50 mL acetone was added KSAc (3.34 g, 29.3 mmol). The reaction was heated to reflux for l6 hrs. The precipitate was removed by filtration and the filtrate was concentrated in vacuo.

The crude product was isolated by a silica gel column using a mixture of EtOAc and hexane (2 to l ratio) as an eluting solvent to give N-Boc-2-pyrrolidinethio-acetate (43% yield).
(M+H)⁺ @ 260.

To a solution of N-Boc-2-pyrrolidinethioacetate (0.9 g, 3.47 mmol) and 6-phthalimido-1-bromohexane (1.08 g, 3.47 mmol) in 10 mL MeOH and 1 mL of DMF was added NaOMe (0.8 mL, 3.47 mmol) under argon at 0°C. After the reaction was stirred at 0°C for 30 minutes, the ice bath was removed; the reaction was stirred at room temperature for 16 hrs and was concentrated in vacuo. The crude product was purified on a silica gel column using EtOAc/Hexane (1 to 4 ratio) to give title compound (35 % yield). 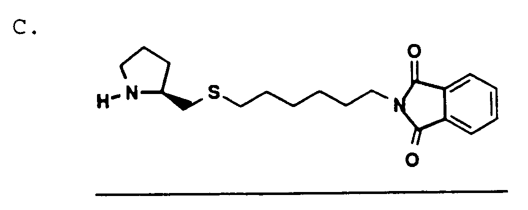
To a solution of N-Boc-2-pyrrolidine thio-6-hexaphthalimide (0.5 g, 11.2 mmol) in 3 mL of CH₂Cl₂ was added 4N HCl (3.5 mL, 3.5 mmol) at 0°C. The ice bath was removed and the reaction was stirred at room temperature for 1 hr. The reaction mixture was concentrated in vacuo to give the title HCl salt (89% yield). 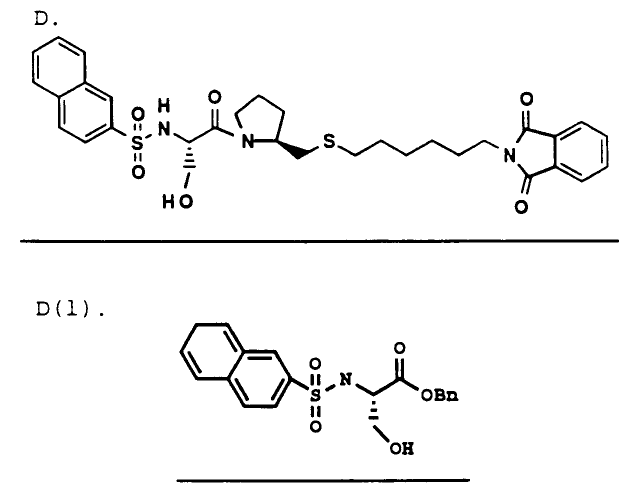
To a solution of 2-naphthalene sulfonyl chloride (3 g, l3.23 mmol) and serine benzylester (3.07 g, l3.23 mmol) in l20 mL CH₂Cl₂ was added Et₃N (5.53 mL, l3.23 mmol) at 0°C. The reaction was stirred at room temperature for l hr and washed with a sat. solution of KHSO₄, brine over Na₂SO₄. The organic layer was concentrated in vacuo to give title compound (93% yield). (M+H)⁺ @ 386.

### D(2). 2-Naphthalenesulfonyl-serine

To a solution of Part D(l) compound (l g, 2.59 mmol) and Pd/C (l0%, 50 mg) in 25 mL EtOAc was added 2.6 mL of lN HCl, followed by H₂. The reaction was stirred at room temperature for l6 hrs and the catalyst was removed by filtration. The filtrate was concentrated in vacuo to give 2-naphthalene sulfonylserine (89% yield). (M+H)⁺ @ 296. 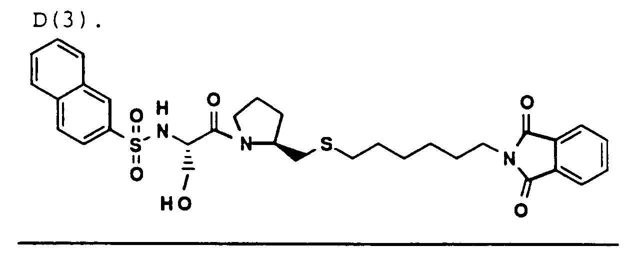
To a solution of 2-naphthalene sulfonyl-serine (293 mg, 0.99 mmol) and HOBT (1.47 g, 1.09 mmol) in 10 mL DMF was added WSC (209 mg, 1.09 mmol) and Part C compound (380 mg, 0.99 mmol), followed by NMM (0.3 mL, 2.18 mmol). The reaction was stirred at room temperature for 16 hrs. The reaction mixture was partitioned between EtOAc and a sat. solution of NaHCO₃ and the organic layer was washed with a sat. solution of KHSO₄, brine, dried over Na₂SO_{4.}, and EtOAc was concentrated in vacuo to give title compound (83 % yield). 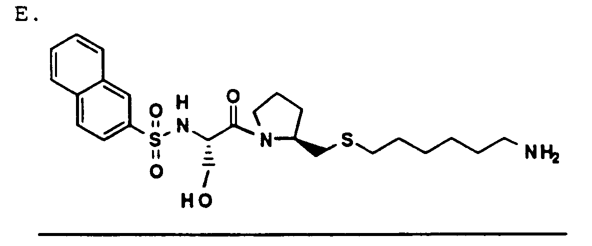
To a solution of Part D compound (510 mg, 0.82 mmol) in 10 mL MeOH was added hydrazine (0.26 mL, 8.2 mmol) at room temperature. The reaction was stirred at 40-50°C for 4 hrs. The reaction mixture was concentrated in vacuo and partitioned between EtOAc and 1N NaOH, dried over Na₂SO₄. The organic layer concentrated in vacuo to give title compound (74 % yield).

### F. [S-(R*,R*)]-N-[2-[[2-[[[6-[(Aminoiminomethyl)amino]hexyl]thio]methyl]-l-pyrrolidinyl]-l-(hydroxymethyl)-2-oxoethyl]-2-naphthalenesulfonamide, trifluoroacetate (l:l) salt

To a solution of Part E amine (300 mg, 0.61 mmol) and amidinesulfonic acid (113 mg, 0.91 mmol) in 10 mL EtOH was added Et₃N (0.26 mL, 1.83 mmol). The reaction stirred at room temperature for 16 hrs. The reaction mixture was concentrated in vacuo and purified by a preparative HPLC to give title compound (34 % yield).

| Elemental Analysis: C₂₅H₃₇N₅O₄S· 1.15TFA · 0.52 H₂O | | | | |
|---|---|---|---|---|
| Calc.: | C, 47.15; | H, 5.71; | N, 10.44; | S 9.56 |
| Found: | C, 47.15; | H, 5.42; | N, 10.36; | S, 9.91. |

[α]_{D} = -91.0 (c = 2.00, CH₃OH).

### Example l3

### [3R-[3R*,3(S*,S*)]]-N-[2-[2-[[[[l-(Aminoiminomethyl)-3-piperidinyl]methyl]thio]methyl]-l-pyrrolidinyl]-l-(hydroxymethyl)-2-oxoethyl]-2-naphthalenesulfonamide, trifluoroacetate (l:l) salt

### A. N-Carbobenzyloxy-3-piperidinemethanol

To a solution of 3(R)-N-carbobenzyloxy-3-carboxypiperidine (2.0 g, 7.6 mmol) in 15 mL THF was added BH₃ · Me₂S dropwise at 0°C and the ice bath was removed. The reaction was stirred at room temperature for 2 hrs. The reaction mixture was quenched with HOAc, MeOH, then 1N 7.6 mL NaOH and was stirred at room temperature for 2 hrs. The reaction mixture was concentrated in vacuo and extracted with EtOAc. The EtOAc layer was washed with 1N NaOH and concentrated in vacuo to give N-carbobenzyloxy-3-piperidinemethanol. 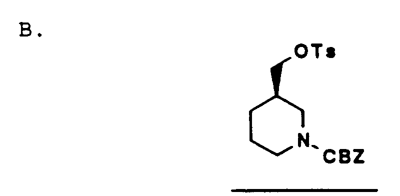
To a solution of Part A compound (2.0 g, 8.03 mmol) and TsCl (3.06 g, 16.06 mmol) in 35 mL CH₂Cl₂ was added Et₃N (3.39 mL, 24.09 mmol) at 0°C. The ice bath was removed and the reaction was stirred at room temperature for 16 hrs. The reaction mixture was washed with H₂O and was concentrated in vacuo to give the crude which was purified by a silica gel column using a mixed solvent (EtOAc : hexane = 1 : 2 ratio) to obtain title compound (70% yield). 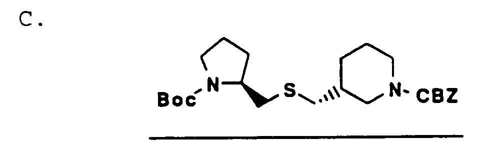
To a solution of N-Boc-2-pyrrolidinethioacetate (Example l2 Part B) (1.0 g, 3.86 mmol) in 30 mL DMF was added NaOMe (0.88mL, 15.44 mmol) under argon at 0°C. The reaction was stirred at 0°C for 5 minutes and Part B compound (1.5 g, 3.86 mmol) was added at 0°C. The ice bath was removed and stirred at room temperature for 2 hrs. The reaction mixture was partitioned between EtOAc and H₂O and the EtOAc was washed with brine, dried over Na₂SO₄, concentrated in vacuo to give title compound (79% yield). 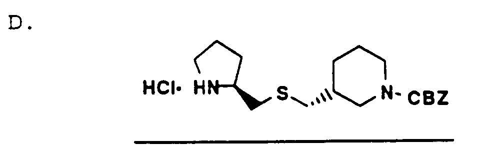
To a solution of Part C compound (1.2 g, 2.46 mmol) in 2 mL CH₂Cl₂ was added 4N HCl (7.4 mL, 7.4 mmol) at 0°C. The ice bath was removed and the reaction was stirred at room temperature for 2 hrs. The reaction was concentrated in vacuo to give title compound. 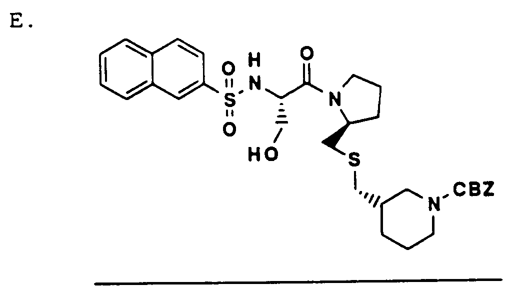
To a solution of 2-naphthalene sulfonyl-serine (prepared in Example l2 Part D) (0.78 g, 2.67 mmol) and HOBT (0.4 g, 2.93 mmol) in 11 mL DMF was added WSC (0.56 g, 2.93 mmol), followed by Part D compound (1.03 g, 2.67 mmol). After 5 minutes, NMM (0.82 mL, 5.86 mmol) was added to adjust the pH to 7.5-8.0 and the reaction stirred at room temperature for 16 hrs. The reaction mixture was partitioned between EtOAc and a sat. solution of NaHCO₃ and the EtOAc layer was washed with a sat. solution of KHSO₄, brine, dried over Na₂SO₄. The organic layer was concentrated in vacuo to give title compound (87 % yield). 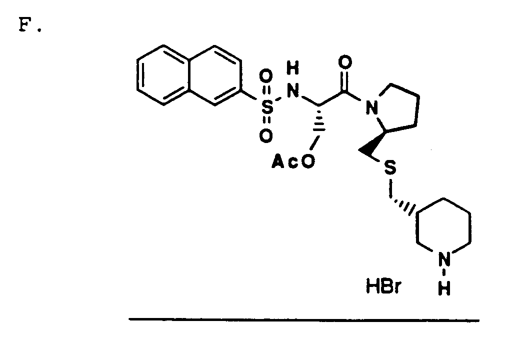
To a solution of Part E compound (1.3 g, 2.08 mmol) in 3 mL CH₂Cl₂ was added HBr (6.3 mL, 6.3 mmol) at 0°C. The ice bath was removed and the reaction was stirred at room temperature for 1 hr. The reaction mixture was concentrated in vacuo to give the title HBr salt (98% yield). 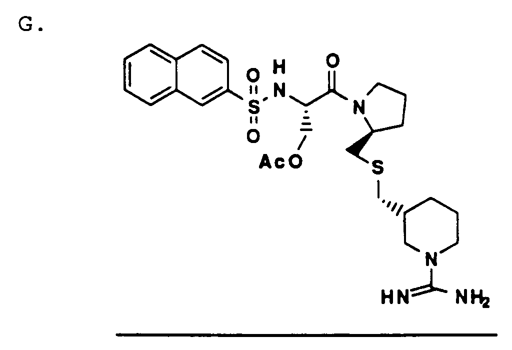
To a solution of the Part F piperidine HBr salt (1.08 g, 2.04 mmol) and H-pyrazole-1-carboxamidine · HCl (490 mg, 3.34 mmol) in 10 mL DMF was added diisopropylethylamine (DIEA) (1.06 mL, 6.08 mmol) and the reaction was stirred at room temperature for 72 hrs. The reaction mixture was purified by a preparative HPLC to give title compound (27 % yield).

### H. [3R-[3R*,3(S*,S*)]]-N-[2-[2-[[[[l-(Aminoiminomethyl)-3-piperidinyl]methyl]thio]methyl]-l-pyrrolidinyl]-l-(hydroxymethyl)-2-oxoethyl]-2-naphthalenesulfonamide, trifluoroacetate (l:l) salt

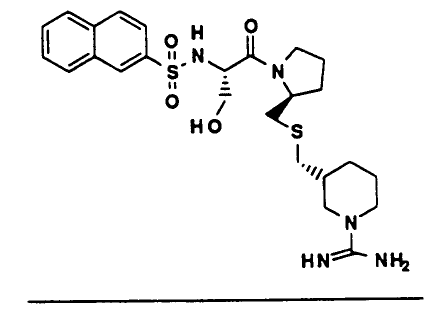

To a solution of Part G compound (0.31 g, 0.54 mmol) in 3 mL, MeOH was added 1N NaOH (1.62 mL, 1.62 mmol) at 0°C. The ice bath was removed and the reaction was stirred at room temperature for 2 hrs. The reaction mixture was concentrated in vacuo and purified by preparative HPLC to give title compound (97 % yield).

| Elemental Analysis: C₂₅H₃₅N₅O₄S₂ · 1.4 TFA · 1.25 H₂O | | | | | |
|---|---|---|---|---|---|
| Calc.: | C, 46.64; | H, 5.48; | N, 9.78; | S, 8.96; | F, 11.15 |
| Found: | C, 46.64; | H, 5.28; | N, 9.77; | S, 8.77; | F, 11.16. |

[α]_{D} = -49.3° (c = 4.1, CD₃OD).

### Example l4

### [3S-[3R*,3(R*,R*)]]-N-[2-[2-[[[[l-(Aminoiminomethyl)-3-piperidinyl]methyl]thio]methyl]-l-pyrrolidinyl]-l-(hydroxymethyl)-2-oxoethyl]-2-naphthalenesulfonamide, trifluoroacetate (l:l) salt

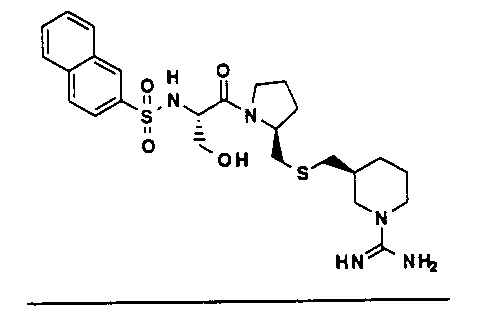

Following the procedure of Example l3, except substituting 3(S)-N-carbobenzyloxy-3-carboxypiperidine for the 3(R) isomer used in Part A of Example l3, the title compound was obtained.

| Elemental Analysis: C₂₅H₃₅N₅O₄S₂ · 1.15TFA · 1.20H₂O | | | | | |
|---|---|---|---|---|---|
| Calc.: | C, 47.77; | H, 5.66; | N, 10.20; | S, 9.34; | F, 9.55 |
| Found: | C, 47.77; | H, 5.37; | N, 10.31; | S, 8.99; | F, 9.57. |

[α]_{D} = -21.0° (c = 4.08, DMSO).

Examples of additional compounds which may be prepared following the procedures set out in Examples l to l4 and in the specification are set out below.

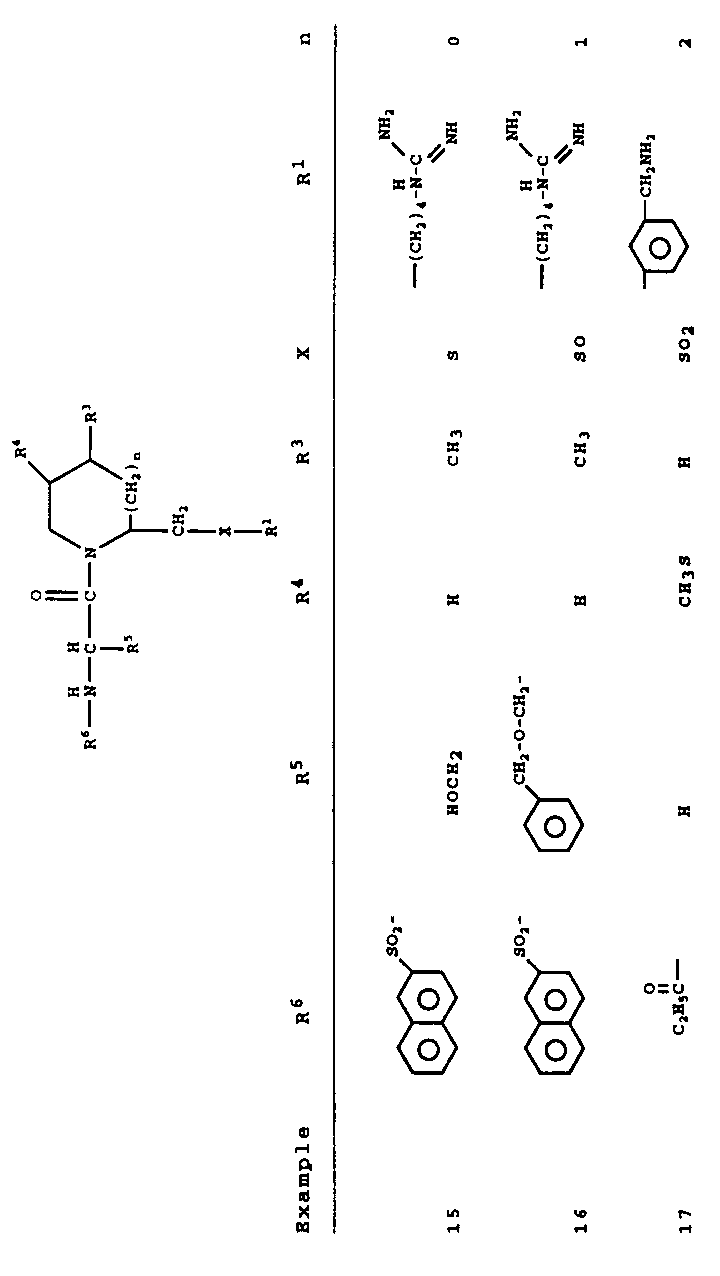

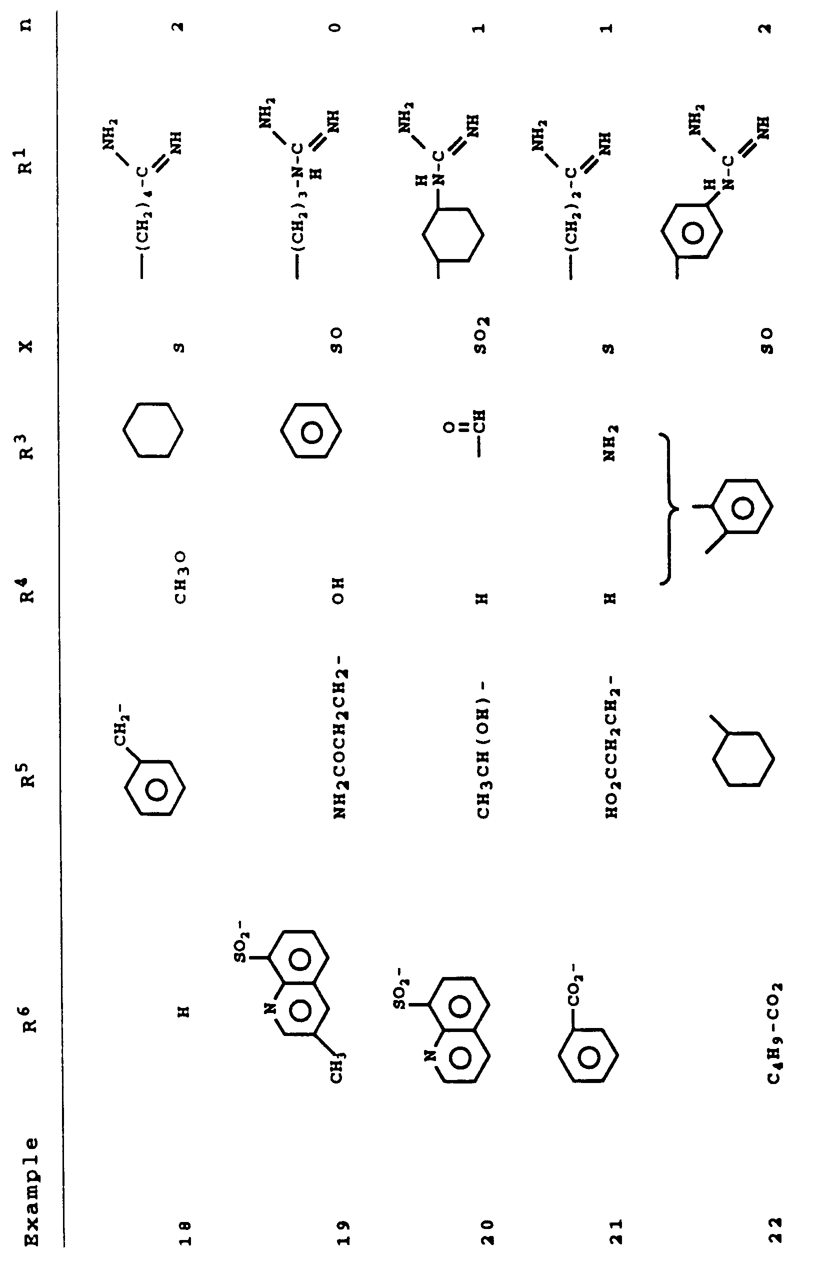

## Claims

1. A compound having the structure 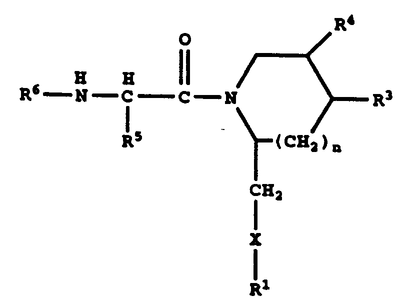 including all stereoisomers, wherein n is 0, l or 2;
X is S, SO, SO₂ or O;
R¹ is -A-R², where A is an alkyl, alkenyl, or alkynyl chain of 2 to 6 carbon atoms and R² is guanidine, amidine, or amino; or
R¹ is -(CH₂)ₚ-A'-R^{2'} where R^{2'} is amidine and A' is an azacycloalkyl ring of 4 to 8 atoms, optionally substituted by alkyl, CO or halo as given by the structure 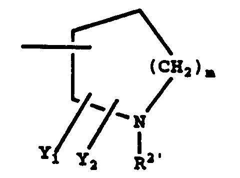 where N and X are separated by at least two carbons;
p is 0, l or 2; m is 0, l, 2, 3 or 4;
Y₁, Y₂ are independently H, alkyl, halo or keto; or
R¹ is -(CH₂)ₚ-A''-R^{2''},
wherein R^{2''} is guanidine, amidine or aminomethyl, and A'' is aryl or cycloalkyl;
R³ and R⁴ are independently hydrogen, lower alkyl, cycloalkyl, aryl, hydroxy, alkoxy, keto, thioketal, thioalkyl, thioaryl, amino or alkylamino, or R³ and R⁴ together with the carbons to which they are attached form a cycloalkyl, aryl or heteroaryl ring;
R⁵ is hydrogen, hydroxyalkyl, aminoalkyl, alkyl, cycloalkyl, aryl, arylalkyl, alkenyl, alkynyl, amidoalkyl, arylalkoxyalkyl or an amino acid side chain, either protected or unprotected; 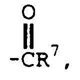
R⁶ is hydrogen, -SO₂R⁷ or -CO₂R⁷ wherein R⁷ is lower alkyl, aryl, cycloheteroalkyl or heteroaryl;
including pharmaceutically acceptable salts thereof.

2. The compound as defined in Claim l wherein R¹ is -A-R².

3. The compound as defined in Claim l wherein R¹ is -(CH₂)ₚ-A'-R^{2'}.

4. The compound as defined in Claim l wherein R¹ is -(CH₂)ₚ-A''-R^{2''}.

5. The compound as defined in Claim l wherein R¹ is -CH₂(CH₂)_{q}-R² and q is l, 2, 3 or 4.

6. The compound as defined in Claim 5 wherein R² is guanidine.

7. The compound as defined in Claim l wherein R¹ is 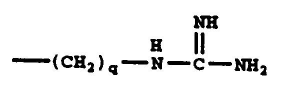 where q is 3 or 4.

8. The compound as defined in Claim l wherein n is O.

9. The compound as defined in Claim l wherein R³ and R⁴ are H.

10. The compound as defined in Claim l wherein R⁶ is -SO₂R⁷.

11. The compound as defined in Claim l wherein R⁷ is

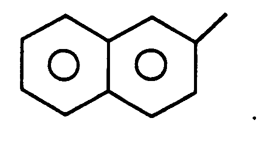

12. The compound as defined in Claim l wherein R⁵ is -CH₂OH or H.

13. The compound as defined in Claim l wherein n is O, X is S or SO₂, R¹ is 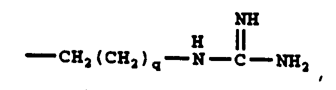 q is 2 or 3, R³ and R⁴ are each H, R⁵ is CH₂OH or H and R⁶ is

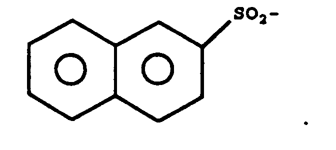

14. The compound as defined in Claim l which is [S-(R*,R*)]-N-[2-[2-[[[4-[(aminoiminomethyl)amino]butyl]thio]methyl]-l-pyrrolidinyl]-l-(hydroxymethyl)-2-oxoethyl]-2-naphthalenesulfonamide, or pharmaceutically acceptable salts thereof or its trifluoroacetate salt;
[S-(R*,R*)]-N-[4-[[[l-(2-amino-l-oxo-3-phenylpropyl)-2-pyrrolidinyl]methyl]thio]butyl]guanidine, or pharmaceutically acceptable salts thereof or its trifluoroacetate salt;
[S-(R*,R*)]-N-[2-[2-[[[4-(aminoiminomethyl)amino]butyl]sulfonyl]methyl]-l-pyrrolidinyl]-l-(hydroxymethyl)-2-oxoethyl]-2-naphthalenesulfonamide, or pharmaceutically acceptable salts thereof or its trifluoroacetate salt;
[S-(R*,R*)]-N-[2-[2-[[[3-[(aminoiminomethyl)amino]propyl]thio]methyl]-l-pyrrolidinyl]-l-(hydroxymethyl)-2-oxoethyl]-2-naphthalenesulfonamide, or pharmaceutically acceptable salts thereof or its trifluoroacetate salt;
[S-(R*,R*)]-N-[2-[2-[[[[l-(aminoiminomethyl)-4-piperidinyl]methyl]thio]methyl]-l-pyrrolidinyl]-l-(hydroxymethyl)-2-oxoethyl]-2-naphthalenesulfonamide, or pharmaceutically acceptable salts thereof or its trifluoroacetate salt;
[S-(R*,R*)]-N-[2-[2-[[[l-(aminoiminomethyl)-4-piperidinyl]methoxy]methyl]-l-pyrrolidinyl]-l-(hydroxymethyl)-2-oxoethyl]-2-naphthalenesulfonamide, or pharmaceutically acceptable salts thereof;
[S-(R*,S*)]-N-[2-[2-[[[4-[(aminoiminomethyl)amino]butyl]thio]methyl]-l-pyrrolidinyl]-2-(hydroxymethyl)-2-oxoethyl]-2-naphthalenesulfonamide or pharmaceutically acceptable salts thereof;
[S-[R*,R*-(E)]]-N-[2-[2-[[(4-amino-2-butenyl)thio]methyl]-l-pyrrolidinyl]-l-(hydroxymethyl)-2-oxoethyl]-2-naphthalenesulfonamide, or pharmaceutically acceptable salts thereof;
[S-[R*,R*-(E)]]-N-[2-[[[4-[(aminoiminomethyl)amino]-2-butenyl]thio]methyl]-l-pyrrolidinyl]-l-(hydroxymethyl)-2-oxoethyl]-2-naphthalenesulfonamide, or pharmaceutically acceptable salts thereof or its trifluoroacetate salt;
[S-(R*,R*)]-N-[2-[2-[[[[l-(aminoiminomethyl)-3-piperidinyl]methyl]thio]methyl-l-pyrrolidinyl]-l-(hydroxymethyl)-2-oxoethyl]-2-naphthalenesulfonamide, or pharmaceutically acceptable salts thereof or its trifluoroacetate salt; or
[S-(R*,R*)]-N-[2-[2-[[[5-[(aminoiminomethyl)amino]pentyl]thio]methyl]-l-pyrrolidinyl]-l-(hydroxymethyl)-2-oxoethyl]-l-naphthalenesulfonamide, or pharmaceutically acceptable salts thereof or its trifluoroacetate salt;
[S-(R*,R*)]-N-[2-[[2-[[[6-[(aminoiminomethyl)amino]hexyl]thio]methyl]-l-pyrrolidinyl]-l-(hydroxymethyl)-2-oxoethyl]-2-naphthalenesulfonamide, or pharmaceutically acceptable salts thereof including its trifluoroacetate salt;
[3R-[3R*,3(S*,S*)]]-N-[2-[2-[[[[l-(aminoiminomethyl)-3-piperidinyl]methyl]thio]methyl]-l-pyrrolidinyl]-l-(hydroxymethyl)-2-oxoethyl]-2-naphthalenesulfonamide, or pharmaceutically acceptable salts thereof including its trifluoroacetate salt;
[3S-[3R*,3(R*,R*)]]-N-[2-[2-[[[[l-(aminoiminomethyl)-3-piperidinyl]methyl]thio]methyl]-l-pyrrrolidinyl]-l-(hydroxymethyl)-2-oxoethyl]-2-naphthalenesulfonamide, or pharmaceutically acceptable salts thereof including its trifluoroacetate salt.

15. The compound as defined in Claim 5 wherein R² is amidinyl or amino.

16. The compound as defined in Claim l wherein R¹ is -A'-R^{2'}.

17. The compound as defined in Claim l6 wherein R^{2'} is aminomethyl.

18. The compound as defined in claim l6 wherein R^{2'} is guanidinyl.

19. The compound as defined in Claim l6 wherein R^{2'} is amidinyl.

20. A pharmaceutical composition comprising a compound as defined in any one of Claims 1-19 and a pharmaceutically acceptable carrier therefor.

21. Use of a compound as defined in any one of Claims 1-19 for the preparation of a pharmaceutical composition for inhibiting or preventing the formation of blood clots.
